# EUROPÄISCHE PATENTSCHRIFT

(11) **EP 1 740 706 B1**
(45) Veröffentlichungstag und Bekanntmachung des Hinweises auf die Patenterteilung: **11.01.2012**
(21) Anmeldenummer: 05745378.9
(22) Anmeldetag: 02.05.2005
(51) Int. Cl.: C12P 3/00, C12P 9/00

(54) **Enzymatisches Verfahren zur Herstellung bioaktiver, Osteoblasten-stimulierender Oberflachen und Verwendung derselben**
Enzymatic method for producing bioactive, osteoblast-stimulating surfaces and use thereof
Procédé enzymatique de production de surfaces bioactives, stimulant les ostéoblastes, et utilisation desdites surfaces

(30) Priorität: 30.04.2004 DE 102004021229
(43) Veröffentlichungstag der Anmeldung: 10.01.2007
(73) Patentinhaber: NanotecMARIN GmbH, 55128 Mainz (DE)
(72) Erfinder: SCHWERTNER, Heiko, 19055 Schwerin (DE); MÜLLER, Werner E.G., 65203 Wiesbaden (DE); SCHRÖDER, Heinz, C., 65189 Wiesbaden (DE)
(74) Vertreter: Krauss, Jan
(86) Internationale Anmeldenummer: PCT/EP2005/004738
(87) Internationale Veröffentlichungsnummer: WO 2005/106004

(56) Entgegenhaltungen:
- WO-A-00/35993
- WO-A-02/10420
- WO-A-2004/033679
- WO-A2-2005/045020
- WO-A2-2005/045020
- TAHIR ET AL.: "Monitoring the formation of biosilica catalysed by histidine-tagged silicatein" CHEMICAL COMMUNICATION, Bd. 24, Dezember 2004 (2004-12), Seiten 2848-2849, XP002344375
- SCHROEDER HEINZ C ET AL: "Mineralization of SaOS-2 cells on enzymatically (sillicatein) modified bioactive osteoblast-stimulating surfaces", JOURNAL OF BIOMEDICAL MATERIALS RESEARCH. PART B: APPLIED BIOMATERIALS, JOHN WILEY & SONS, HOBOKEN, NJ, US, vol. 75, no. 2, 1 November 2005 (2005-11-01), pages 387-392, XP009141546, ISSN: 1552-4973

### SEQUENCE LISTING

<110> Dr. Heiko Schwertner
<120> Enzymatisches Verfahren zur Herstellung bioaktiver,
   Osteoblasten-stimulierender Oberflächen und Verwendung
<130> U30089PCT
<160> 12
<170> PatentIn version 3.2
<210> 1
   <211> 330
   <212> PRT
   <213> suberites domuncula
<400> 1
   Met Leu Val Thr Val Val Val Leu Gly Leu Leu Gly Phe Ala Ser Ala
   1 5 10 15
   Ala Gln Pro Lys Phe Glu Phe Val Glu Glu Trp Gln Leu Trp Lys Ser
   20 25 30
   Thr His Ser Lys Met Tyr Glu Ser Gln Leu Met Glu Leu Glu Arg His
   35 40 45
   Leu Thr Trp Leu Ser Asn Lys Lys Tyr Ile Glu Gln His Asn Val Asn
   50 55 60
   Ser His Ile Phe Gly Phe Thr Leu Ala Met Asn Gln Phe Gly Asp Leu
   65 70 75 80
   Ser Glu Leu Glu Tyr Ala Asn Tyr Leu Gly Gln Tyr Arg Ile Glu Asp
   85 90 95
   Lys Lys Ser Gly Asn Tyr Ser Lys Thr Phe Gln Arg Asp Pro Leu Gln
   100 105 110
   Asp Tyr Pro Glu Ala Val Asp Trp Arg Thr Lys Gly Ala Val Thr Ala
   115 120 125
   Val Lys Asp Gln Gly Asp Cys Gly Ala Ser Tyr Ala Phe Ser Ala Met
   130 135 140
   Gly Ala Leu Glu Gly Ala Asn Ala Leu Ala Lys Gly Asn Ala Val Ser
   145 150 155 160
   Leu Ser Glu Gln Asn Ile Ile Asp Cys Ser Ile Pro Tyr Gly Asn His
   Gly Cys His Gly Gly Asn Met Tyr Asp Ala Phe Leu Tyr Val Ile Ala
   180 185 190
   Asn Glu Gly Val Asp Gln Asp Ser Ala Tyr Pro Phe Val Gly Lys Gln
   195 200 205
   Ser Ser Cys Asn Tyr Asn Ser Lys Tyr Lys Gly Thr Ser Met Ser Gly
   210 215 220
   Met Val Ser Ile Lys Ser Gly Ser Glu Ser Asp Leu Gln Ala Ala Val
   225 230 235 240
   Ser Asn Val Gly Pro Val Ser Val Ala Ile Asp Gly Ala Asn Ser Ala
   245 250 255
   Phe Arg Phe Tyr Tyr Ser Gly Val Tyr Asp Ser Ser Arg Cys Ser Ser
   260 265 270
   Ser Ser Leu Asn His Ala Met Val Val Thr Gly Tyr Gly Ser Tyr Asn
   275 280 285
   Gly Lys Lys Tyr Trp Leu Ala Lys Asn Ser Trp Gly Thr Asn Trp Gly
   290 295 300
   Asn Ser Gly Tyr Val Met Met Ala Arg Asn Lys Tyr Asn Gln Cys Gly
   305 310 315 320
   Ile Ala Thr Asp Ala Ser Tyr Pro Thr Leu
   325 330
<210> 2
   <211> 1214
   <212> DNA
   <213> Suberites domuncula
<400> 2
   aggatagaaa gtctacaatc tgtaaggaca atgcttgtca cagtggtagt actgggtcta      60
   ctggggtttg cttctgcagc ccagcccaag tttgaatttg tagaagaatg gcagctgtgg     120
   aagtccactc actctaagat gtacgagtca cagttaatgg aactcgaaag acatctgacg     180
   tggctctcca ataagaaata tatcgagcaa cacaatgtca actcacacat tttcggtttt     240
   actctggcaa tgaaccagtt tggagatctg agtgaattgg agtatgctaa ctatcttggc     300
   cagtatcgca ttgaggataa aaaatctggc aactactcaa agacttttca gcgtgatcct     360
   ctacaggact accctgaagc tgtagactgg agaaccaaag gagctgtcac ggctgtcaag     420
   gaccagggag actgtggtgc tagctatgct ttcagtgcta tgggtgcttt ggagggtgct     480
   aatgctttag ccaagggaaa tgcagtatct ctcagtgaac agaacatcat tgattgctcg     540
   attccttacg gtaaccacgg ttgtcatgga ggcaatatgt atgatgcttt tttgtatgtc     600
   atcgctaacg agggggtcga tcaggacagt gcatatccat ttgtaggaaa gcaatccagc     660
   tgcaactata atagtaaata caaaggtaca tcaatgtcgg ggatggtgtc aatcaaaagt     720
   ggtagtgagt ctgacttaca agcagctgtt tcaaacgttg gccctgtatc tgttgctatt     780
   gatggtgcta acagtgcctt caggttttac tacagtggtg tctatgactc atcacgatgc     840
   tctagtagta gtcttaacca cgcaatggta gtcactggat acggatcata caatgggaaa     900
   aaatactggc tggccaagaa tagctgggga actaactggg gtaacagtgg ctatgtgatg     960
   atggctcgca acaagtacaa ccagtgtgga attgctaccg atgcatctta tcccacccta    1020
   taaacttata tatatatagt cttagaaaca ttatcctttt ctttaccctt gtctctatag    1080
   gccatagagt gattgtaggc tgtttgcatt tgatgactgt atatacccta tcattttttg    1140
   tgattctatc tgattaaaaa tcccataccc gaccaaacca tcaatttatc aaatcatgaa    1200
   aaaaaaaaaa aaaa                                                      1214
<210> 3
   <211> 383
   <212> PRT
   <213> Suberites domuncula
<400> 3
   Met Ser Ala Leu Lys Phe Val Val Ala Leu Cys Val Val His Thr Ser
   1               5                   10                  15
   Leu Gly Ile Ala Glu Ser Val Gly Lys Ser Lys Thr Ala Gly Leu Ser
               20                  25                  30
   Asp Asp Gly Asn Tyr Thr Ala Val Thr Lys Ser Val Arg Leu Thr Pro
           35                  40                  45
   Val Leu Glu Phe Glu Glu Asp Trp Lys Gln Trp Thr Thr Asp His His
       50                  55                  60
   Lys Val Tyr Ser Asp Val Arg Glu Arg Val Asp Lys Tyr Thr Val Trp
   65                  70                  75                  80
   Arg Ala Asn Lys Glu Tyr Ile Asp Gln His Asn Gln Asn Ala Gln Arg
                   85                  90                  95
   Leu Gly Tyr Thr Leu Lys Met Asn Lys Phe Gly Asp Leu Thr Thr Lys
               100                 105                 110
   Glu Phe Ile Glu Gly Tyr His Cys Val Gln Asp Tyr Gln Pro Thr Asn
   115 120 125
   Ala Ser His Leu Asn Lys Lys His Lys Thr His Ala Phe Val Asp Tyr
   130 135 140
   Gly Asp Phe Val Arg Gly Gly Thr Gly Glu Gly Val Arg Gly Val Gly
   145 150 155 160
   Asn Met Pro Glu Thr Met Asp Trp Arg Thr Ser Gly Val Val Thr Lys
   165 170 175
   Val Lys Asp Gln Leu Arg Cys Gly Ser Ser Tyr Ala Phe Ser Ala Met
   180 185 190
   Ala Ser Leu Glu Gly Ile Asn Ala Leu Ser Tyr Gly Ser Leu Val Thr
   195 200 205
   Leu Ser Glu Gln Asn Ile Val Asp Cys Ser Val Thr Tyr Gly Asn His
   210 215 220
   Gly Cys Ala Cys Gly Asp Val Asn Arg Ala Leu Leu Tyr Val Ile Glu
   225 230 235 240
   Asn Asp Gly Val Asp Thr Trp Lys Gly Tyr Pro Ser Gly Gly Asp Pro
   245 250 255
   Tyr Arg Ser Lys Gln Tyr Ser Cys Lys Tyr Glu Arg Gln Tyr Arg Gly
   260 265 270
   Ala Ser Ala Arg Gly Ile Val Ser Leu Ala Ser Gly Asp Glu Asn Thr
   275 280 285
   Leu Leu Thr Ala Val Ala Asn Ser Gly Pro Val Ser Val Tyr Val Asp
   290 295 300
   Ala Thr Ser Thr Ser Phe Gln Phe Tyr Ser Asp Gly Val Leu Asn Val
   305 310 315 320
   Pro Tyr Cys Ser Ser Ser Thr Leu Ser His Ala Leu Val Val Ile Gly
   325 330 335
   Tyr Gly Lys Tyr Ser Gly Gln Asp Tyr Trp Leu Val Lys Asn Ser Trp
   340 345 350
   Gly Pro Asn Trp Gly Val Arg Gly Tyr Gly Lys Leu Ala Arg Asn Lys
   355 360 365
   Gly Asn Lys Cys Gly Ile Ala Thr Ala Ala Ser Phe Pro Thr Leu
       370                 375                 380
<210> 4
   <211> 1372
   <212> DNA
   <213> Suberites domuncula
<400> 4
   acttagtata ttatggtagt gtacaatacc tatctccata gaggcatgca taactaggtt      60
   tattgaatag ctgctggcac aatttgttct caagttggtg ctattagatt tgtgttctag     120
   aatgtcagca ttgaagtttg tagttgcctt gtgtgtagtt cacacaagct taggaatagc     180
   tgagtcagtt ggtaagagca agactgcagg cctaagtgac gatggcaact acacagctgt     240
   caccaaatct gtaagactga ctccagttct agagtttgag gaagattgga agcaatggac     300
   aactgatcat cacaaggtct actctgatgt gagggagaga gtggacaagt acactgtatg     360
   gagagctaat aaagagtaca ttgatcaaca caaccagaac gcacagagat tgggatacac     420
   actcaaaatg aacaaatttg gagatttgac taccaaggag ttcattgaag gctatcactg     480
   tgttcaggac taccaaccta ccaatgcatc acatttgaat aagaaacaca aaacgcacgc     540
   gtttgtcgac tatggtgact ttgtgagggg tggaactggt gagggtgtga ggggtgtagg     600
   aaacatgccg gagactatgg actggagaac ttctggagtt gtcacaaaag ttaaagatca     660
   gcttcgttgt ggtagcagct atgcgttctc tgccatggct tcattggaag gaataaatgc     720
   tctttcctac ggatctttgg tgacactcag tgaacaaaac attgtagact gctcggttac     780
   ctatggcaac catggttgcg cctgtggtga tgtaaaccgt gctctactgt atgtgataga     840
   gaatgatggc gttgacacgt ggaagggtta tccttctggt ggggatcctt atcgatcaaa     900
   gcaatactct tgcaaatacg agagacagta tcgtggggcc tctgctagag gtatagtcag     960
   tctagctagt ggtgatgaga acacattgtt gacagcagta gctaactctg gaccagtgag    1020
   tgtgtatgtg gacgctactt caacatcctt ccagttttac agtgatggag tgttgaatgt    1080
   tccctattgc tcctctagca cgctgagtca tgccttggtt gtcattggtt acgggaagta    1140
   cagcggacaa gattactggc ttgttaaaaa cagctggggt cctaactggg gagtgcgggg    1200
   ctatgggaag ttggcaagaa acaagggcaa caaatgtgga atagccacag cggctagttt    1260
   cccaacatta tgacacttta gttgatcaaa caattaatca taaattatta caacatgtag    1320
   tataatgatg cccccccatt gctcaatagc ttatctttga acaagaaaaa aa            1372
<210> 5
   <211> 379
   <212> PRT
   <213> Suberites domuncula
<400> 5
   Met Ser Ala Ile Leu Lys Arg Asn Val Pro Ile Gln Arg Val Gly Leu
   1               5                   10                  15
   Pro Leu Thr Ser Tyr Val Ser Arg Trp Ala Ser Ala Leu Pro Thr Arg
               20                  25                  30
   Thr His Pro Phe Tyr Lys Leu Val Asp Asp Ser Thr Thr Pro Val Thr
           35                  40                  45
   Arg Ser Thr Leu Leu Ser Ala His Met Val Asp Thr Leu Leu Asp Glu
       50                  55                  60
   Asn Gln Gln Ser Arg His Glu Asn Gln His Thr Asp Thr Ser Tyr Lys
   65                  70                  75                  80
   Met Tyr Gln Gly Leu Lys Phe Val Val Lys Thr Leu Phe Thr Pro Ser
                   85                  90                  95
   Lys Cys His Arg His Phe Ser Thr Ser Ala His Leu Ser Ala Met Gly
               100                 105                 110
   Arg His Gln Ser Pro Ile Asn Ile Ile Thr Ser Ser Thr Thr Lys Gly
           115                 120                 125
   Pro Ser Leu Lys Pro Leu Lys Phe Ser Lys Ser Trp Asp Lys Pro Val
       130                 135                 140
   Ile Gly Thr Val Lys Asp Thr Gly Tyr Tyr Leu Lys Phe Ala Pro Glu
   145                 150                 155                 160
   Ser Ala Ala Glu Lys Cys Thr Leu His Thr Tyr Asn Gly Glu Tyr Ile
                   165                 170                 175
   Leu Asp His Phe His Tyr His Trp Gly Lys Lys Asp Gly Glu Gly Ala
               180                 185                 190
   Glu His Phe Ile Asp Gly Lys Gln Tyr Asp Ile Glu Phe His Phe Val
           195                 200                 205
   His Lys Lys Val Gly Leu Thr Asp Pro Asp Ala Arg Asp Ala Phe Ala
       210                 215                 220
   Val Leu Gly Val Phe Gly Lys Ala Asp Pro Arg Leu Lys Ile Asn Gly
   225                 230                 235                 240
   Ile Trp Glu Leu Leu Ser Pro Ser Thr Val Leu Thr Val Asp Ser Thr
                   245                 250                 255
   Arg Asn Val Ala Asp Val Val Pro Ser Lys Leu Leu Pro Ser Ala Arg
               260                 265                 270
   Asp Tyr Phe His Tyr Glu Gly Ser Leu Thr Thr Pro Thr Tyr Gly Glu
           275                 280                 285
   Val Val His Trp Phe Val Leu Asn Glu Pro Ile Ala Val Pro Ser Glu
       290                 295                 300
   Tyr Leu Ser Ala Leu Arg Gln Met Gln Ala Asp Lys Glu Gly Thr Val
   305                 310                 315                     320
   Ile Asp Ser Asn Tyr Arg Glu Leu Gln Glu Val His Asn Arg Pro Val
                   325                 330                 335
   Gln Arg Phe Lys Ser Asp Glu Gln Gly Arg Gly Glu Phe Asp Asp Ile
               340                 345                 350
   Ser Lys Asn Glu Asp Ile Val Glu Asp Leu Ser Lys Leu Ser Gly Asn
           355                 360                 365
   Phe Ile Arg Glu Leu Val Arg Lys Ile Tyr Trp
       370                 375
<210> 6
   <211> 1405
   <212> DNA
   <213> Suberites domuncula
<400> 6
   gaattcggca cgagggacaa ctttgcataa cttttactgt ccatgtttaa cgtttagatc     60
   tagtactagt agtctacaag aacaactgtc aacaactgtc agattatgtg tataaaccaa    120
   gatgtctgca attcttaaga gaaacgtacc tatccaaaga gtcggtctcc cactgacctc    180
   ctatgtcagt agatgggctt ctgctctgcc caccaggacc catccttttt acaagttggt    240
   tgatgacagt accaccccag tgacaaggtc tactcttctc agtgctcata tggttgacac    300
   cttgctagat gagaaccagc agagcagaca tgaaaaccaa cacacagaca cgtcttacaa    360
   aatgtaccag ggattaaaat ttgttgtaaa gacgctgttt actccatcga aatgccaccg    420
   tcacttctcc acatcagctc atttgtctgc catgggtcga catcaatccc ccatcaatat    480
   aatcacctcc agtacgacca aaggaccgtc attgaaaccg ttaaaattta gcaagagttg    540
   ggacaagcca gtaatcggca ccgtcaaaga tactggctat tatcttaaat ttgcaccaga    600
   atctgcagca gagaagtgca cattgcatac gtacaatggt gaatatatcc tagatcattt    660
   ccattatcac tgggggaaga aggatgggga aggagcagag catttcatcg atggaaaaca    720
   atacgacatc gagttccact ttgtacataa aaaggttggg ttgactgatc cagatgctag    780
   agacgctttt gctgttttgg gcgtttttgg aaaggccgac cctcgtttga agatcaatgg    840
   aatctgggag ctactctcac cgtcaactgt cctgactgtc gactcaacac gaaacgtcgc    900
   tgatgttgtt ccctctaagc ttctcccaag tgccagagac tattttcact atgaaggttc    960
   tttgaccaca cctacgtatg gtgaggttgt gcactggttt gttctcaatg aacccatagc   1020
   tgtccctagt gagtatctgt cagctctgag acagatgcaa gctgacaaag aaggtactgt   1080
   gattgactca aactatcgag agcttcaaga agtccacaat cgacctgtgc aacgatttaa   1140
   gagtgatgag caagggagag gagaatttga cgatatttct aagaatgagg acattgtgga   1200
   ggacttgtct aaattgtctg gtaactttat tagagagctg gtcaggaaga tatattggtg   1260
   accttttcct acacttgtta gagttttagg ccagaataca tttcatcatt tggactgtta   1320
   ttttgtgtac actgcttagc agtttatata aacactacaa tgccattatt ataatatagc   1380
   caatgctgtg atttgaaaaa aaaaa                                         1405
<210> 7
   <211> 268
   <212> PRT
   <213> Suberites domuncula
<400> 7
   Met Val Ala Ile Ser Gly Gln Ala Ile Gly Pro Gln Gly Pro Arg Gly
   1               5                   10                  15
   Leu Pro Gly Arg Asp Gly Arg Asp Gly Gln Pro Gly Ser Pro Gly Ser
               20                  25                  30
   Asp Gly Gln Ser Gly Ser Pro Gly Arg Asp Gly Arg Asp Gly Leu Thr
           35                  40                  45
   Gly Pro Gln Gly Val Ile Gly Glu Pro Gly Pro Ala Asn Gly Pro Gln
       50                  55                  60
   Gly Pro Arg Gly Leu Gln Gly Thr Arg Gly Glu Pro Gly Ser Gln Gly
   65                  70                  75                  80
   Pro Ala Gly Gln Pro Gly Pro Thr Ser Gly Gly Val Val Tyr Thr Arg
                   85                  90                  95
   Trp Gly Lys Ser Thr Cys Pro Ser Val Ala Gly Thr Gln Leu Val Tyr
               100                 105                 110
   Ala Gly Arg Ala Gly Gly Ser Glu His Arg Asp Ala Gly Ala Ala Asn
           115                 120                 125
   His Leu Cys Met Pro Leu Asp Pro Glu Tyr Thr Leu Gln His Arg Ser
       130                 135                 140
   Gly Val Gln Gly Ser Met Tyr Val Tyr Gly Thr Glu Tyr Gln Ser Pro
   145                 150                 155                 160
   Ile Arg Gly Thr Asp Asn His Asn Val Pro Cys Ala Val Cys Ser Thr
                   165                 170                 175
   Ser Thr Arg Ala Gln Leu Leu Met Ile Pro Ala Lys Thr Ser Cys Pro
               180                 185                 190
   Thr Ser Trp Thr Arg Glu Tyr Tyr Gly Tyr Leu Met Ser Gln His Arg
           195                 200                 205
   Ser His His Pro Ser Met Tyr Glu Cys Val Asp Lys Ile Lys Asn Leu
       210                 215                 220
   Ser Gln Gly Val Leu Leu Thr Pro Met Val Leu Phe Ser Ile His Val
   225                 230                 235                 240
   Glu Ala Thr Cys Asn Gly Met Gln Cys Pro Pro Tyr Asn Asn Tyr Lys
                   245                 250                 255
   Glu Leu Asn Cys Val Val Cys Thr Lys Arg Ser Phe
               260                 265
<210> 8
   <211> 1038
   <212> DNA
   <213> Suberites domuncula
<400> 8
   ggcacgaggt taaacagctc tcgtgaaaaa catggaacca aagacctact actctttgct     60
   gcagttacac tactagtaat gattgtggca atatcaggtc aggctatagg acctcaaggc    120
   ccacgtggac taccaggaag agatggtcgt gatggacaac ctgggtcacc tggtagtgat    180
   ggacaatctg gatcacctgg tagagatgga agagatggat tgactggccc tcaaggtgta    240
   ataggagaac caggaccagc caatggacca caaggaccca gaggactaca ggggacaagg    300
   ggagaaccag ggtcacaagg acctgctggt caaccaggac ccacgagtgg aggtgttgtc    360
   tacacaaggt gggggaagag cacttgtcct agtgttgctg ggactcaact ggtatatgct    420
   ggaagagctg gcgggagtga acacagagat gctggggcag ccaaccatct ctgtatgcct    480
   ttagaccctg agtacaccct acaacatcgt agtggagtgc aaggcagtat gtatgtatat    540
   ggaacagaat accaatctcc tattagagga acagataatc acaacgttcc atgtgctgtg    600
   tgttcaacat ctactcgagc ccagctctta atgattccag ctaagacatc ctgtccgaca    660
   tcatggacta gagagtacta tggctatctc atgtctcaac atcgttccca tcatccttca    720
   atgtatgaat gcgttgacaa gatcaagaat ctgtcccagg gagttctgct aacaccaatg    780
   gtgctctttt ctatccacgt tgaagctacc tgcaatggta tgcaatgtcc tccatacaat    840
   aactacaaag aactgaactg tgttgtctgc acaaagtaaa cttattgaca tagtttgtat    900
   tgcgtgcatg cagtcattaa tgtgataggt actgccagtg tgaaaataga gattattgtg    960
   ggtgtgtgcg tgtgcgtgtt ttgtgcatgt gtgctgtttt tcaatatatc ttttattaat   1020
   ataaaaaaaa aaaaaaaa                                                 1038
<210> 9
   <211> 30
   <212> RNA
   <213> Suberites domuncula
<400> 9
   accaggacac ccgaagcgag acggagaacc 30
<210> 10
   <211> 40
   <212> DNA
   <213> Suberites domuncula
<400> 10
   tattctagaa ttatagggtg ggataagatg catcggtagc 40
<210> 11
   <211> 39
   <212> DNA
   <213> Suberites domuncula
<400> 11
   tatccatggt ggcaatatca ggtcaggcta taggacctc 39
<210> 12
   <211> 38
   <212> DNA
   <213> Suberites domuncula
<400> 12
   tataagcttc gctttgtgca gacaacacag ttcagttc 38

## Beschreibung

### 1. Stand der Technik

Siliciumdioxid, Silikate und Silicone sind vielbenutzte und wirtschaftlich bedeutende Materialien in der Industrie. Sie gehören auch zu den Hauptmaterialien, die zur Herstellung von Hochtechnologieprodukten (wie optischer und mikroelektronischer Instrumente, Herstellung von Nanopartikeln) verwendet werden. Siliciumdioxid (SiO₂) kommt in kristalliner und in amorpher Form vor. Amorphes SiO₂ wird u. a. als Molekülsieb, als Katalysator, Füllstoff, Weißmittel, zur Adsorption, als Carrier, Stabilisator oder Träger für Katalysatoren benutzt. Amorphes SiO₂ ("Biosilica") ist auch das Material, aus dem die durch Biomineralisation gebildeten Skelettstrukturen vieler ein- und vielzelliger Organismen bestehen, wie die Schalen von Kieselalgen (Diatomeen) und die Nadeln (Spicula) von Kieselschwämmen.

Die chemische Synthese von polymeren Silicaten erfordert üblicherweise drastische Bedingungen wie hoher Druck und hohe Temperatur. Kieselschwämme sind dagegen befähigt, mit Hilfe spezifischer Enzyme Silicatgerüste unter milden Bedingungen, d. h. bei relativ niedriger Temperatur und niedrigem Druck, zu bilden. Die SiO₂-Synthese bei diesen Organismen ist durch hohe Spezifität, Regulierbarkeit und die Fähigkeit zur Synthese definierter Nanostrukturen ausgezeichnet.

In den letzten Jahren konnten erste Einblicke in die Mechanismen, die an der Bildung von biogenem Silica beteiligt sind, erhalten werden. Überaschenderweise stellte sich heraus, dass Kieselschwämme in der Lage sind, ihr Silica-Skelett enzymatisch zu synthetisieren. Dies wurde durch die Isolierung der ersten Gene und Proteine deutlich, die an der Siliciumdioxid-Bildung beteiligt sind.

Die Spicula-Bildung in Demospongien beginnt um ein axiales Filament, welches aus einem Protein ("Silicatein") besteht, das enzymatisch aktiv ist und die Synthese von amorphem Siliciumdioxid vermittelt (Cha et al. (1999) Proc Natl Acad Sci USA 96:361-365; Krasko et al. (2000) Europ J Biochem 267:4878-4887). Das Enzym wurde aus dem marinen Kieselschwamm *Suberites domuncula* kloniert (Krasko et al. (2000) Europ J Biochem 267:4878-4887) und seine technische Verwendung beschrieben; bei dem zuerst beschriebenen Enzym handelt es sich um Silicatein-α (auch lediglich Silicatein genannt) WO 00/35993. Methods, compositions, and biomimetic catalysts, such as silicateins and block copolypeptides, used to catalyze and spatially direct the polycondensation of silicon alkoxides, metal alkoxides, and their organic conjugates to make silica, polysiloxanes, polymetallo-oxanes, and mixed poly(silicon/ metallo)oxane materials under environmentally benign conditions. Inventors/ Applicants: Morse DE, Stucky GD, Deming, TD, Cha J, Shimizu K, Zhou Y; DE 10037270 A 1. Silicatein-vermittelte Synthese von amorphen Silicaten und Siloxanen und ihre Verwendung. Deutsches Patentamt 2000. Anmelder und Erfinder: Müller WEG, Lorenz B, Krasko A, Schröder HC; WO 02/10420. Silicateinmediated synthesis of amorphous silicates and siloxanes and use thereof. Inventors/Applicants: Müller WEG, Lorenz B, Krasko A, Schröder HC). Es ist in der Lage, Biosilica aus organischen Siliciumverbindungen (Alkoxysilanen) zu synthetisieren.

Neben dem Silicatein-α wurde auch das Silicatein-β kloniert **(**DE 103 52 433.9. Enzymatische Synthese, Modifikation und Abbau von Silicium(IV)- und anderer Metall(IV)-Verbindungen. Deutsches Patentamt 2003. Anmelder: Johannes Gutenberg-Universität Mainz; Erfinder: Müller WEG, Schwertner H, Schröder HC).

Die Silicateine sind Vertreter der Cathepsin-Familie. Ebenso wie bei den Cathepsinen z. B. aus höheren Vertebraten sind die drei Aminosäuren Cys, His und Asn, welche die katalytische Triade (CT) der Cysteinproteasen bilden, in den Schwamm-Cathepsinen (abgeleitete Aminosäuresequenzen der Cathepsin L-cDNAs der Schwämme *Geodia cydonium* und *S*. *domuncula*) vorhanden; beim Silicatein-α und Silicatein-β *(S. domuncula*) ist jedoch der Cysteinrest durch Serin ersetzt (Krasko et al. (2000) Europ J Biochem 267:4878-4887).

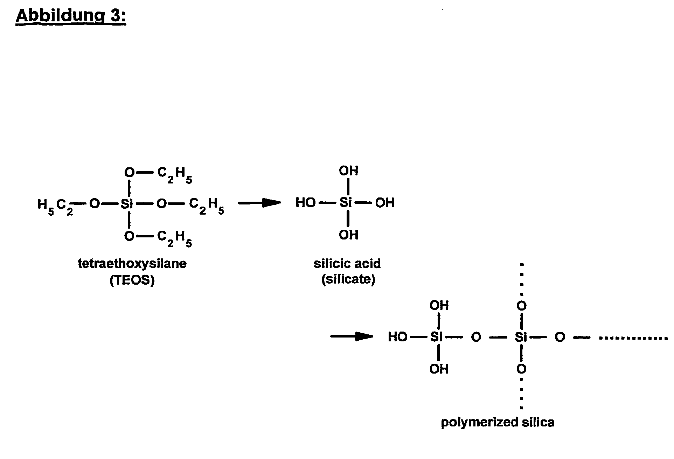

Zur Messung der enzymatischen Aktivität der rekombinanten Silicateine wird üblicherweise Tetraethoxysilan als Substrat benutzt, wobei das nach der enzymvermittelten Abspaltung von Ethanol entstehende Silanol polymerisiert (Figur 3). Die Menge an polymerisiertem Siliciumdioxid kann mit Hilfe eines Molybdat-Assays bestimmt werden (Cha et al. (1999) Proc Natl Acad Sci USA 96:361-365; Krasko et al. (2000) Europ J Biochem 267:4878-4887).

Ebenfalls gelang es, aus *S*. *domuncula* ein Enzym zu klonieren, das in der Lage ist, amorphes Siliciumdioxid aufzulösen (Schröder HC, Krasko A, Le Pennec G, Adell T, Wiens M, Hassanein H, Müller IM, Müller WEG (2003) Silicase, an enzyme which degrades biogenous amorphous silica: Contribution to the metabolism of silica deposition in the demosponge Suberites domuncula. Progr Molec Subcell Biol 33 :250-268; DE 102 46 186.4. Abbau und Modifizierung von Silicaten und Siliconen durch Silicase und Verwendung des reversiblen Enzyms. Deutsches Patentamt 2002. Anmelder: Johannes Gutenberg-Universität Mainz; Erfinder: Müller WEG, Krasko A, Schröder HC). Das Silica-abbauende Enzym, Silicase, wurde unter Anwendung der Technik des "Differential Display der mRNA" identifiziert. Die Silicase codiert die für ein Carboanhydrase-ähnliches Enzym. Die rekombinante Silicase bewirkt die Auflösung von Siliciumdioxid unter Bildung von freier Kieselsäure. Das Enzym ist aber auch in der reversiblen Reaktion zu deren Synthese in der Lage. Northern-Blot-Experimente ergaben, dass in *S*. *domuncula* bei Erhöhung der Konzentration von Silicium im Medium sowohl die Expression des Silica-anabolen Enzyms, des Silicateins, als auch diejenigen des Silica-katabolen Enzyms, der Silicase, ansteigt.

### 1.1. Osteoblasten

Osteoblasten sind Knochen-bildende Zellen. Sie synthetisieren und sezernieren die meisten Proteine der Knochenmatrix, einschließlich Typ I Kollagen und Nichtkollagen-Proteine. Sie besitzen einen hohe Gehalt an alkalischer Phosphatase, die an der Mineralisation beteiligt ist. Osteoblasten sprechen auf 1α,25-Dihydroxyvitamin D₃ [1,25(OH)₂D₃]_{,} Glucocorticoide und Wachstumsfaktoren an. 1,25(OH)₂D₃ ist ein Stimulator der Knochenresorption; es erhöht in reifen Osteoblasten die Expression von Genen wie Osteocalcin, die mit dem Mineralisationprozess zusammenhängen.

Typische Marker für den Osteoblasten-Phänotyp sind u. a. die alkalische Phosphatase, Osteocalcin, Typ I Kollagen, Fibronectin, Osteonectin, Sialoprotein, Proteoglycane und Kollagenase. Die alkalische Phosphatase ist ein Ektoenzym (ein von der Zelle nach außen orientiertes Enzym), das über einen Glycosylphosphatidylinositol-Anker an die Membran gebunden ist.

Es existieren eine Reihe von Osteoblasten-Zelllinien. SaOS-2-Zellen sind eine etablierte humane Osteosarkom-Zelllinie, die als experimentelles Modell zum Studium der Osteoblastenfunktion benutzt wird. Unter den verfügbaren humanen Zelllinien sind sie vermutlich die am höchsten differenzierten Osteoblasten-ähnlichen Zellen (Rifas et al. (1994) Endocrinology 134:213-221). SaOS-2-Zellen besitzen eine hohe alkalische Phosphatase-Aktivität, Osteonectin sowie Parathormon- und 1,25(OH)₂D₃-Rezeptoren und sind zur Mineralisierung in der Lage (Rodan et al. (1987) Cancer Res 47:4961-4966; McQuillan et al. (1995) Bone 16: 415-426). Das zum Aufbau der Matrix synthetisierte Kollagen besteht hauptsächlich aus Typ I und Typ V Kollagen.

Die Mineralisierung von Osteoblasten-Kulturen wie SaOS-2 wird durch Zusatz von β-Glycerophosphat gefördert. β-Glycerophosphat wird von der nach außen orientierten alkalischen Phosphatase gespalten, wobei anorganisches Phosphat (Pᵢ) freigesetzt wird. Häufig wird zur Mineralisierung auch Ascorbinsäure beigefügt, um die Bildung der Kollagenmatrix zu fördern, auf die sich die Hydroxylapatit-Kristalle ablagern können (McQuillan et al. (1995) Bone 16:415-426). Bei stimulierten SaOS-2-Zellen lässt sich die Mineralisierung 6 bis 7 Tage nach Konfluenz gut nachweisen.

Der Mechanismus der Osteoblasten-Adhäsion an die extrazelluläre Matrix des Knochens ist komplex. Die Adhäsion an das Kollagensubstrat scheint die Osteoblasten-Differenzierung und -Funktion zu regulieren. Zum Beispiel blockieren Peptide, die das Arg-Gly-Asp (RGD)-Motiv enthalten, die Mineralisierung und anschließende Osteoklasten-Entwicklung in Ratten-Osteoblasten, haben aber keinen Einfluss auf die Kollagen-Synthese durch diese Zellen (Gronowicz und Derome (1994) J Bone Miner Res 9:193-201). Andererseits wurde gezeigt, dass Oberflächen mit RGD-Tripeptiden die Osteoblasten-Aktivität fördern (EI-Ghannam et al. (2004) J Biomed Mater Res 68A:615-627).

Wechselwirkungen von Integrinen mit extrazellulären Matrix-Proteinen sind am Mechanismus der Adhäsion und der sich anschließenden zellulären Prozesse entscheidend beteiligt. Humane Osteoblasten exprimieren eine Vielzahl von Integrinen. Es wurde gezeigt, dass bestimmte Integrine eine Rolle bei der Induktion der Expression der alkalischen Phosphatase durch Interleukin-1 in humanen MG-63 OsteosarcomZellen spielen (Dedhar (1989) Exp Cell Res 183:207-214). Andere Integrine wurden als Adhäsionsrezeptoren für Kollagen identifiziert (Hynes (1992) Cell 69:11-25). So wird das im Typ I Kollagen enthaltene Tetrapeptid-Motiv Asp-Gly-Glu-Ala (DGEA) (Staatz et al. (1991) J Biol Chem 266:7363-7367) von einem Integrin, das von humanen Osteoblasten exprimiert wird, erkannt (Clover et al. (1992) J Cell Sci 103:267-271). Das DGEA-Peptid bewirkt auch einen Ca²⁺-Anstieg in SaOS-2-Zellen (McCann et al. (1997) Matrix Biol. 16:271-280).

### 2. Gegenstand der Erfindung

Es besteht ein großer Bedarf an alternativen Knochenersatzmaterialien aufgrund der Nachteile von Autotransplantaten, die gegenwärtig in der Klinik für die Knochenreparatur und den Knochenersatz bevorzugt benutzt werden. In der Orthopädie werden häufig bioabbaubare Polymere wie Polylactide (PLA), Polyglycolide (PGA) und ihre Co-Polymere (PLAGA) benutzt. In den letzten Jahren wurden sogenannte bioaktive Materialien wie 45S5 bioaktives Glas entwickelt, welche die Neubildung von Knochen stimulieren und eine kontinuierliche Verbindung zum Knochen über eine Calciumphosphat-Schicht an ihrer Oberfläche aufbauen (Hench et al. (1991) J Amer Cerm Soc 74:1487). Hierdurch wird jedoch eine nicht-physiologische Oberflächen-Matrix (Glas-Oberfläche) bereitgestellt.

Es ist somit eine Aufgabe der vorliegenden Erfindung ein Verfahren zur Herstellung einer, geeigneten physiologischen Oberfläche zur Verfügung zu stellen, die gegenüber den herkömmlich verwendeten Materialien verbesserte Eigenschaften aufweist.

Diese Aufgabe wird gemäß eines ersten Aspekts der Erfindung durch Bereitstellung eines Verfahrens gemäß Anspruch 1 gelöst, welches die Verwendung von physiologischen Molekülen/ Molekülaggregaten (Kollagen) und mit enzymatisch hergestelltem Biosilica modifizierten Oberflächen-Matrices umfasst.

Im Rahmen der Erfindung wird als "bioaktiv" ein Material bezeichnet, wenn an seiner Oberfläche eine spezifische biologische Antwort hervorgerufen wird, die letztendlich zur Ausbildung einer (stabilen) Bindung zwischen dem Material und dem Gewebe (wie z. B. Knochenneubildung) führt. Ein "bioaktives" Material trägt somit zur Förderung von Zellwachstum und/oder -Differenzierung und/oder Modulation von spezifischen Zellfunktionen (wie Förderung der Mineralisierung durch Osteoblasten oder der Förderung der Kollagenbildung durch Fibroblasten und/oder weiteren Zellfunktionen) bei.

Es wurde gezeigt, dass durch Oberflächen-aktive Gläser (bioaktive Gläser) die Expression von Kollagen Typ I, der alkalischen Phosphatase sowie des Knochen morphogenetischen Proteins-2 (BMP-2) in SaOS-2-Osteoblasten *in vitro* erhöht wird (Gao et al. (2001) Biomaterials 22:1475-1483; Bosetti et al. (2003) J Biomed Mater Res 64A:189-95).

Kieselsäure spielt eine wichtige Rolle bei der Knochenbildung. So ist bekannt, dass Orthokieselsäure die Typ 1 Kollagen-Synthese und die Differenzierung in humanen Osteoblasten *in vitro* stimuliert (Reffitt et al. (2003) Bone 32:127-135). Ebenfalls werden auch die Alkalische Phosphatase-Aktivität und Osteocalcin signifikant erhöht.

Als Übersichtsartikel über klinische Anwendungen von bioaktiven Gläsern und Glas-Keramiken werden angegeben: Gross et al. (1988) CRC Critical Reviews in Biocompatibility 4:2; Yamamuro et al. (Hrsg.), Handbook on Bioactive Ceramics, Vol I: Bioactive Glasses and Glass-Ceramics, Vol. II. CRC Press, Boca Raton, FL, 1990; Hench und Wilson (1984) Science 226:630.

Voraussetzungen für derartige Knochenersatzmaterialien sind, dass sie biokompatibei, bioabbaubar und osteokonduktiv (zur Unterstützung des Knochenwachstums fähig), d. h. bioaktiv sind (zur Bildung einer Calciumphosphat-Schicht auf ihrer Oberfläche fähig sind, siehe oben).

Gemäß einem weiteren Aspekt der vorliegenden Erfindung wird somit ein Verfahren zur Herstellung bioaktiver Oberflächen durch enzymatische Modifikation von Molekülen oder Molekülaggregaten auf Oberflächen mit amorphem Siliciumdioxid (Silica) beschrieben, wobei zur enzymatischen Modifikation ein Polypeptid eingesetzt wird, dadurch gekennzeichnet, dass das Polypeptid eine tierische, bakterielle, pflanzliche oder Pilz Silicatein-α- oder Silicatein-β-Domäne umfasst, die mindestens 50%, bevorzugt mindestens 75% und am meisten bevorzugt mindestens 95% Sequenzidentität zu der in SEQ ID Nr. 1 bzw. SEQ ID Nr. 3 gezeigten Sequenz aufweist.

Bisher war nicht bekannt und nicht aus dem Stand der Technik zu erkennen, dass es möglich ist, durch enzymatische Modifikation von Molekülen oder Molekülaggregaten auf Oberflächen mit amorphem Siliciumdioxid (Silica) bioaktive Oberflächen zu erhalten.

Es wird somit weiterhin ein erfindungsgemäßes Verfahren zur Verfügung gestellt, das dadurch gekennzeichnet ist, dass als Substrat zur enzymatischen Modifikation Verbindungen wie Kieselsäuren, Monoalkoxysilantriole, Dialkoxysilandiole, Trialkoxysilanole oder Tetraalkoxysilane eingesetzt werden.

Gemäß einem weiteren Aspekt der vorliegenden Erfindung kann das Verfahren auch zur Herstellung bioaktiver Oberflächen durch enzymatische Modifikation von Molekülen oder Molekülaggregaten auf Oberflächen mit Siliconen dienen, wobei zur enzymatischen Modifikation ebenfalls ein Polypeptid eingesetzt wird, das dadurch gekennzeichnet ist, dass es eine tierische, bakterielle, pflanzliche oder Pilz Silicatein-α- oder Silicatein-β-Domäne umfaßt, die mindestens 50%, bevorzugt mindestens 75% und am meisten bevorzugt mindestens 95% Sequenzidentität zu der in SEQ ID Nr. 1 bzw. SEQ ID Nr. 3 gezeigten Sequenz aufweist.

Für den letztgenannten Aspekt (Herstellung bioaktiver Oberflächen durch enzymatische Modifikation von Molekülen oder Molekülaggregaten auf Oberflächen mit Siliconen) können als Substrat zur enzymatischen Modifikation Verbindungen wie Monoalkoxysilandiole, Monoalkoxysilanole, Dialkoxysilanole, Alkyl-, Aryl- oder Metallo-Silantriole, Alkyl-, Aryl- oder Metallo-Silandiole, Alkyl-, Aryl- oder Metallo-Silanole, Alkyl-, Aryl- oder Metallo-Monoalkoxysilandiole, Alkyl-, Aryl- oder Metallo-Monoalkoxysilanole, Alkyl-, Aryl- oder Metallo-Dialkoxysilanole, Alkyl-, Aryl- oder Metallo-Trialkoxysilane eingesetzt werden.

Es kann weiterhin ein Verfahren auch zur Herstellung bioaktiver Oberflächen durch enzymatische Modifikation von Molekülen oder Molekülaggregaten auf Oberflächen mit amorphem Siliciumdioxid (Silica) dienen, wobei zur enzymatischen Modifikation ein Polypeptid eingesetzt wird, das dadurch gekennzeichnet ist, dass es eine tierische, bakterielle, pflanzliche oder Pilz Silicase-Domäne umfasst, die mindestens 25%, bevorzugt mindestens 50%, weiter bevorzugt mindestens 75% und am meisten bevorzugt mindestens 95% Sequenzidentität zu der in SEQ ID Nr. 5 gezeigten Sequenz aufweist.

Gemäß einem weiteren Aspekt der vorliegenden Erfindung kann durch das erfindungsgemäße Verfahren eine Herstellung bioaktiver Oberflächen durch enzymatische Modifikation von Molekülen oder Molekülaggregaten auf Oberflächen von Glas, Metallen, Metalloxiden, Kunststoffen, Biopolymeren oder anderen Materialien erfolgen.

Gemäß Anspruch 1 wird ein Verfahren zur Herstellung bioaktiver Oberflächen durch enzymatische Modifikation von Molekülen oder Molekülaggregaten auf Oberflächen zur Verfügung gestellt, wobei es sich bei den Molekülen oder Molekülaggregaten um Kollagen handelt. Bevorzugt handelt es sich um Kollagen aus einem Meeresschwamm.

Dabei wird ein Verfahren zur Unterstützung des Wachstums, der Aktivität und/oder der Mineralisierung von Zellen/Zellkulturen, insbesondere Osteoblasten, zur Verfügung gestellt, wobei a) Moleküle oder Molekülaggregate auf Oberflächen mit amorphem Siliciumdioxid (Silica) enzymatisch modifiziert werden und b) zur enzymatischen Modifikation ein Polypeptid eingesetzt wird, das dadurch gekennzeichnet ist, dass das Polypeptid eine tierische, bakterielle, pflanzliche oder Pilz Silicatein-α- oder Silicatein-β-Domäne umfasst, die mindestens 50%, bevorzugt mindestens 75% und am meisten bevorzugt mindestens 95% Sequenzidentität zu der in SEQ ID Nr. 1 bzw. SEQ ID Nr. 3 gezeigten Sequenz aufweist.

Bei einem Verfahren zur Unterstützung des Wachstums, der Aktivität und/oder der Mineralisierung von Zellen/Zellkulturen, insbesondere Osteoblasten, kann zur enzymatischen Modifikation von Oberflächen mit amorphem Siliciumdioxid (Silica) auch ein Polypeptid eingesetzt werden, das dadurch gekennzeichnet ist, dass das Polypeptid eine tierische, bakterielle, pflanzliche oder Pilz Silicase-Domäne umfasst, die mindestens 25%, bevorzugt mindestens 50%, weiter bevorzugt mindestens 75% und am meisten bevorzugt mindestens 95% Sequenzidentität zu der in SEQ ID Nr. 5 gezeigten Sequenz aufweist.

Das vorstehend beschriebene erfindungsgemäße Verfahren wird in der Zellkultur, beim Tissue Engineering oder bei der Herstellung von medizinischen Implantaten eingesetzt.

Weiterchin beschrieben wird eine Kieselsäureenthaltende Struktur oder Oberfläche, die nach dem erfindungsgemäßen Verfahren erhalten wurde.

Das erfindungsgemäß verwendete Polypeptid (Silicatein-α- oder Silicatein-β aus *Suberites domuncula* gemäß SEQ ID Nr. 1 bzw. SEQ ID Nr. 3 oder ein dazu homologes Polypeptid, das in der Aminosäuresequenz der Silicatein-α- oder Silicatein-β-Domäne mindestens 50%, bevorzugt mindestens 75% und am meisten bevorzugt mindestens 95% Sequenzidentität zu der in SEQ ID Nr. 1 bzw. SEQ ID Nr. 3 gezeigten Sequenz besitzt) kann, neben der natürlichen Form, ferner dadurch gekennzeichnet sein, dass es synthetisch hergestellt worden ist oder dass das Polypeptid in einem prokaryotischen oder eukaryotischen Zellextrakt oder -lysat vorliegt. Der Zellextrakt oder das Lysat kann aus einer Zelle *ex vivo* oder *ex vitro* gewonnen werden, zum Beispiel einer rekombinanten bakteriellen Zelle oder einem Meeresschwamm. Bei dem verwendeten Polypeptid kann es sich auch um eine Silicase aus *Suberites domuncula* gemäß SEQ ID Nr. 5 oder ein dazu homologes Polypeptid, das in der Aminosäuresequenz der Silicase-Domäne mindestens 25%, bevorzugt mindestens 50%, weiter bevorzugt mindestens 75% und am meisten bevorzugt mindestens 95% Sequenzidentität zu der in SEQ ID Nr. 5 gezeigten Sequenz besitzt, handeln.

Das erfindungsgemäß verwendete Polypeptid kann nach herkömmlichen im Stand der Technik bekannten Verfahren gereinigt werden und somit im wesentlichen frei von anderen Proteinen vorliegen.

Die Eigenschaften der für das Silicatein-α-Polypeptid und das Silicatein-β-Polypeptid aus *S*. *domuncula* codierende cDNAs sowie die aus der Nucleotidsequenz abgeleiteten Polypeptide wurden beschrieben (WO 00/35993; DE 10037270 A 1; WO 02/10420; DE 103 52 433.9). Das Molekulargewicht des rekombinanten Silicatein-α-Polypeptids ist ∼28,5 kDa (∼26 kDa Silicatein plus 2 kDa Vektor); der isoelektrische Punkt liegt bei pl 6,16.

Ebenfalls wurden die Eigenschaften der für die Silicase aus *S*. *domuncula* codierende cDNA sowie das aus der Nucleotidsequenz abgeleitete Polypeptid beschrieben (DE 102 46 186.4).

In den beigefügten Figuren und den SEQ IDs zeigen:
SEQ ID No. 1: Die Aminosäuresequenz des erfindungsgemäß verwendeten Silicatein-α- Polypeptids aus *S*. *domuncula.*
SEQ ID No. 2: Die Nukleinsäuresequenz der cDNA des erfindungsgemäß verwendeten Silicatein-α- Polypeptids aus *S*. *domuncula.*
SEQ ID No. 3: Die Aminosäuresequenz des erfindungsgemäß verwendeten Silicateins-β aus *S*. *domuncula* (SIA_SUBDO).
SEQ ID No. 4: Die Nukleinsäuresequenz des erfindungsgemäß verwendeten Silicateins-β aus *S*. *domuncula.*
SEQ ID No. 5: Die Aminosäuresequenz des verwendeten Silicase aus *S*. *domuncula.*
SEQ ID No. 6: Die Nukleinsäuresequenz der CDNA der verwendeten Silicase aus *S*. *domuncula.*
SEQ ID No. 7: Die Aminosäuresequenz des erfindungsgemäß verwendeten Kollagens 3 aus *S*. *domuncula* (SIA_SUBDO).
SEQ ID No. 8: Die Nukleinsäuresequenz des erfindungsgemäß verwendeten Kollagens 3 aus *S*. *domuncula.*

**Figur 1****:**
   Expression von Siiicatein-α von *S*. *domuncula.* Nucleotidsequenz des Silicatein-α-Klons *(S. domuncula)* sowie "Forward Primer" und "Reverse Primer" zur Amplifizierung der für die kurze Silicatein-α-Form kodierenden cDNA zur Klonierung in den Expressionsvektor pBAD/gIII-A und aus der Nucleotidsequenz abgeleitete Aminosäuresequenz des rekombinanten Proteins (kurze Form des Silicatein-α).
**Figur 2****:**
   Expression von nichtfibrillärem Typ 3 Kollagen von *S*. *domuncula.* Nucleotidsequenz des Typ 3 Kollagen-Klons *(S. domuncula)* sowie "Forward Primer" Col3_f und "Reverse Primer" Col 3_r zur Amplifizierung der für Typ 3 Kollagen kodierenden cDNA zur Klonierung in den Expressionsvektor pBAD/gIII-A (die Restriktionsstellen von *N*coI und *Hin*dIII sind unterstrichen) und aus der Nucleotidsequenz abgeleitete Aminosäuresequenz des rekombinanten Proteins.
**Figur 3****:**
   Bestimmung der Silicatein-Aktivität. Als Substrat wird üblicherweise Tetraethoxysilan (TEOS) benutzt.
**Figur 4****:**
   Stimulierung der Mineralisierung von SaOS-2-Zellen nach Coaten der Kulturplatten mit rekombinanten nichtfibrillären Schwamm-Kollagen (Typ 3; *S*. *domuncula)* im Vergleich zu fibrillärem Typ 1 Rind-Kollagen (Sigma). Die Kulturplatten (24-Well-Platten) wurden mit verschiedenen Mengen (10 µg/ml oder 30 µg/ml) an entweder rekombinantem Typ 3 Kollagen *(S. domuncula)* oder Typ 1 Kollagen (Rind; Firma Sigma) gecoated. Danach wurden die SaOS-2-Zellen auf den Platten ausgesät und für 2 und 12 Tage unter Standardbedingungen kultiviert. β-Glycerophosphat (β-GP; 10 mM) wurde am Tag 7 zu den Ansätzen hinzugefügt. Danach wurde die Mineralisierung mit Alizarin Red-S (AR-S; **A**) sowie die Gesamt-DNA (**B**) bestimmt. In (**C**) ist die Mineralisierung in nmol Alizarin Red/µg Gesamt-DNA angegeben.
**Figur 5****:**
   Wachstum von SaOS-2-Zellen auf der erfindungsgemäß enzymatisch modifizierten, Osteoblasten-stimulierenden Oberfläche im Vergleich zu Kontrolloberflächen (Zelldichte). Gezeigt sind die Ergebnisse, die mit SaOS-2-Zellen erhalten wurden, die in Wells auf einer nicht-modifizierten Oberfläche wuchsen (= Kontrolle) (O), sowie von SaOS-2-Zellen, die auf folgendermaßen modifizierten Oberflächen wuchsen: (a) Modifikation durch Coaten mit rekombinantem Typ 3 Kollagen *(S. domuncula)* und enzymatisch synthetisiertem Biosilica (mittels Silicatein-(α und TEOS) (■), (b) Modifikation durch Coaten mit rekombinantem Rinder-Typ 1-Kollagen und enzymatisch synthetisiertem Biosilica (mittels Silicatein-α und TEOS) (▲), (c) Modifikation mit rekombinantem Typ 3 Kollagen alleine *(S. domuncula)* (Δ), (d) Modifikation mit rekombinantem Rinder-Typ 1-Kollagen alleine (◊), (e) Modifikation mit Silicatein alleine (●) und (f) Modifikation durch Behandlung mit TEOS ohne Zusatz eines Proteins (Kollagen oder Silicatein) (□). β-Glycerophosphat (10 mM) wurde am Tag 7 zu den Ansätzen hinzugefügt. Angegeben ist die Zelldichte (Zellen pro cm²) am Tag 1, 2, 3, 4, 6 und 8 nach dem Umsetzen der Zellen.
**Figur 6****:**
   Gesamt-DNA-Menge von SaOS-2-Zell-Kulturen auf der erfindungsgemäß enzymatisch modifizierten, Osteoblasten-stimulierenden Oberfläche im Vergleich zu nicht modifizierten Kontrolloberflächen. Die Zellen wuchsen in Wells, deren Oberflächen entweder mit rekombinantem Typ 3 Kollagen *(S. domuncula)* oder Typ 1 Kollagen (Sigma), beide gecoated mit enzymatisch synthetisiertem Biosilica (mittels Silicatein-α [Si] und TEOS) oder nicht modifiziert wurden. β-Glycerophosphat (10 mM) wurde am Tag 7 zu den Ansätzen hinzugefügt. Angegeben ist die Menge an Gesamt-DNA pro Kultur (Well) am Tag 1, 2, 3, 4, 6 und 8 nach dem Umsetzen der Zellen.
**Figur 7**:
   Mineralisierung von SaOS-2-Zellen auf der erfindungsgemäß enzymatisch modifizierten, Osteoblasten-stimulierenden Oberfläche im Vergleich zu nicht modifizierten Kontrolloberflächen. Der Nachweis der Mineralisierung erfolge am Tag 12 mit Alizarin Red-S. β-Glycerophosphat (10 mM) wurde am Tag 7 zu den Ansätzen hinzugefügt. **1A**: SaOS-2-Zellen, gewachsen auf nicht-modifizierter Oberfläche, mit Zusatz von β-Glycerophosphat ab Tag 7 (relative Stärke der Mineralisierung: ++). **1B, 1C**: SaOS-2-Zellen, gewachsen auf nicht-modifizierter Oberfläche, ohne Zusatz von β-Glycerophosphat (Kontrolle; relative Stärke der Mineralisierung: +). **2A**, **2B,** 2C: Sa-OS-2-Zellen, gewachsen auf modifizierter Oberfläche (Modifikation durch Coaten mit rekombinantem Schwamm-Kollagen Typ 3 und enzymatisch - mittels Silicatein-α und TEOS - synthetisiertem Biosilica), mit Zusatz von β-Glycerophosphat (relative Stärke der Mineralisierung: +++). **3A**, **3B, 3C**: SaOS-2-Zellen mit Zusatz von β-Glycerophosphat, gewachsen auf modifizierter Oberfläche (Modifikation durch Coaten mit Rinder-Typ 1-Kollagen und enzymatisch - mittels Silicatein-α und TEOS - synthetisiertem Biosilica) (relative Stärke der Mineralisierung: +++).
**Abbildung 8****:**
   Stimulierung der Mineralisierung von SaOS-2-Zellen, die auf der erfindungsgemäß enzymatisch modifizierten Oberfläche wuchsen, im Vergleich zu SaOS-2-Zellen auf Oberflächen nach Coaten mit Kollagen alleine und Kontrollen (nicht-gecoatete Platten ohne und mit β-Glycerophosphat). Zum Coaten der Kulturplatten wurde entweder Typ 1 Kollagen (Rind; Sigma) alleine bzw. rekombinantes nichtfibrilläres Typ 3 Kollagen *(S. domuncula)* alleine oder Typ 1 Kollagen plus Silicatein-α plus TEOS (Synthese von Biosilica-modifiziertem Rinder-Kollagen) bzw. rekombinantes Typ 3 Kollagen plus Silicatein-α plus TEOS (Synthese von Biosilica-modifiziertem Schwamm-Kollagen) verwendet. Danach wurden die SaOS-2-Zellen auf den Platten ausgesät und für 2 und 12 Tage unter Standardbedingungen kultiviert. β-Glycerophosphat (β-GP; 10 mM) wurde am Tag 7 zu den Ansätzen hinzugefügt. Die Mineralisierung ist in nmol Alizarin Red/µg Gesamt-DNA angegeben.

### Beispiele

### 2.1. Mineralisierung von SaOS-2-Zellen an enzymafisch modifizierten Oberflächen

Für die folgenden Versuche wurden humane Osteoblastenzellen (SaOS-2-Zellen) benutzt. SaOS-2-Zellen stammen von einem osteogenem Sarkom (McQuillan et al. (1995) Bone 16:415-426). Für alle Kulturen wurde das Zellwachstum sowie die Mineralisierung bestimmt. Als weiterer Differenzierungsmarker wurde neben der Mineralisierung auch die Expression der alkalischen Phosphatase gemessen.

Die SaOS-2-Zellen wurden mit 10 mM β-Glycerophosphat, das am Tag 7 nach dem Umsetzen der Zellen (Start der experimentellen Kulturen) hinzugefügt wurde, für bis zu 12 Tage kultiviert. Danach wurden in den Ansätzen mit Alizarin Red S die Menge an Calciumphosphat-Ablagerungen bestimmt. Die Ergebnisse wurden auf die Gesamt-DNA bezogen.

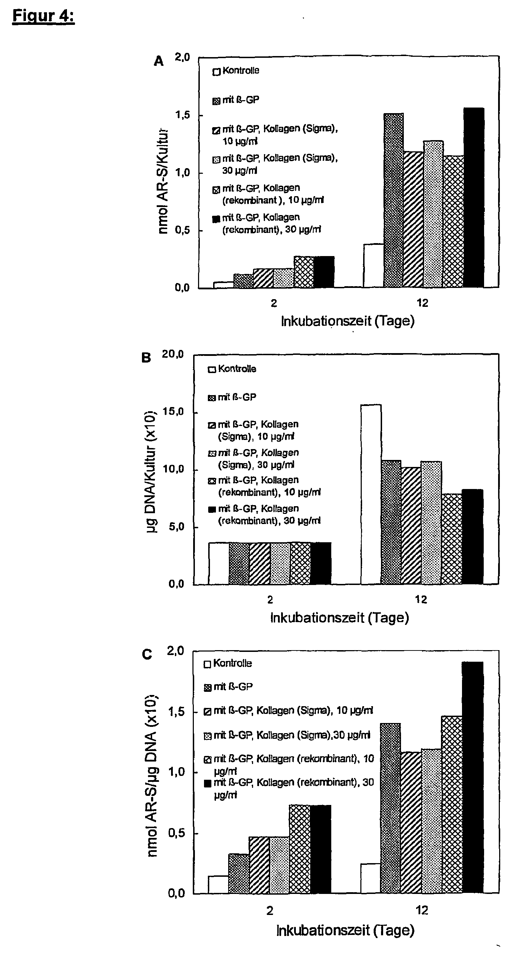

Die Mineralisierung der SaOS-2-Zellen wird durch Coaten der Kulturplatten mit Kollagen stark stimuliert (Figur 4). Das rekombinante Typ 3 Schwamm-Kollagen (*S*. *domuncula)* war dabei effizienter als Typ 1 Rinder-Kollagen (Sigma) (sowohl bei einer Inkubationszeit von 2 Tagen als auch 12 Tagen, wenn die Messwerte auf µg DNA pro Ansatz bezogen wurden). Erst nach einer längeren Inkubationsperiode (12 Tage) war die Stimulierung der Mineralisierung in den Ansätzen mit β-Glycerophosphat etwa gleich derjenigen in den mit dem Typ 1 Rinder-Kollagen gecoateten Wells. Für die mit dem rekombinanten Schwamm-Kollagen gecoateten Wells lag jedoch auch zu diesem Zeitpunkt die Mineralisierung größer als in allen anderen Ansätzen.

Das Coaten der Platten (Wells) mit Kollagen (sowohl Typ 1 Rinder-Kollagen als auch rekombinantes Typ 3 Schwamm-Kollagen) hatte jedoch einen negativen Einfluss auf das Wachstum der SaOS-2-Zellen (angegeben als µg DNA pro Ansatz) bei einer längeren Inkubationsperiode (12 Tage; Figur 4).

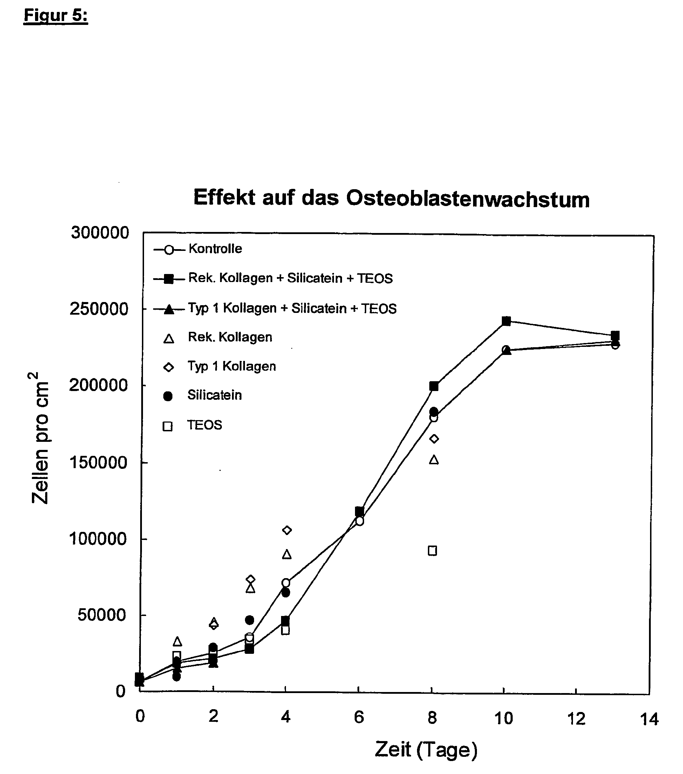

Analoge Ergebnisse wurden erhalten, wenn die Zelldichte (Zellen pro cm²) bestimmt wurde (Figur 5). Zwar wurde bei kürzeren Inkubationsperioden (1 bis 4 Tage) eine deutliche Stimulierung des Wachstums der Zellen in den Wells (Ansätzen) gefunden, die mit Kollagen (Typ 1 Rinder-Kollagen oder rekombinantes Typ 3 Schwamm-Kollagen) gecoated worden waren, aber bei einer längeren Inkubationsperiode (8 Tage) lag das Wachstum unter den Kontrollwerten (Wells ohne Kollagen-Coating).

Im Gegensatz hierzu wurden in den Wells, deren Oberflächen mit dem erfindungsgemäßen Verfahren behandelt worden waren (Modifizierung der Oberfläche mit Kollagen plus enzymatisch - mittels Silicatein und TEOS - hergestelltem Kollagen) höhere Zelldichten, d. h. ein besseres Wachstum, als in den Kontrollen gefunden (Figur 5).

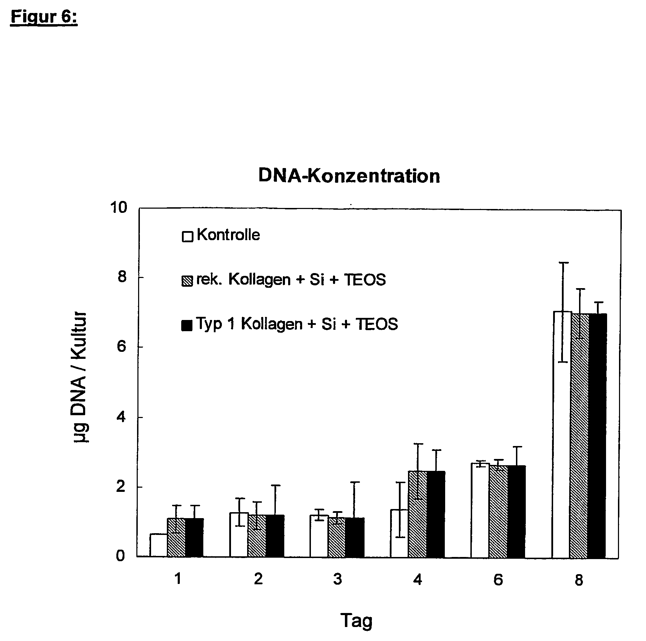

Auch die Bestimmung der DNA-Konzentration ergab, dass in den Wells, deren Oberflächen mit dem erfindungsgemäßen Verfahren behandelt worden waren, keine Herabsetzung des Zellwachstums (basierend auf dem Wert für die Gesamt-DNA pro Kultur) auftrat. Am Tag 4 war die Gesamt-DNA in den behandelten (modifizierten) Wells sogar noch höher als bei der Kontrolle (Figur 6).

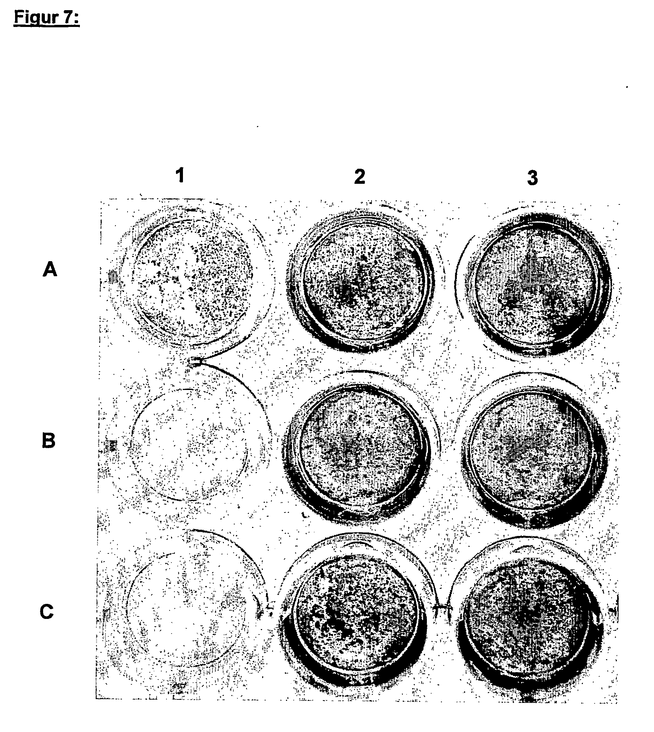

Die drastischsten Unterschiede zwischen der erfindungsgemäß enzymatisch modifizierten, Osteoblasten-stimulierenden Oberfläche im Vergleich zu nicht modifizierten Kontrolloberflächen zeigten sich bei der Bestimmung der Mineralisierung (Calciumphosphat-Ablagerung) der SaOS-2-Zellen. Figur 7 zeigt den Nachweis der Mineralisierung am Tag 12 mit Alizarin Red-S. Auf den Kontroll-Oberflächen der nicht-modifizierter Wells wurde nach Zugabe von β-Glycerophosphat (an Tag 7) nur ein vergleichsweise schwacher Anstieg der Mineralisierung (Well-Nr. 1A) gegenüber den Kontrollen ohne β-Glycerophosphat (Weil-Nr. 1 B und 1 C). Dagegen wurde bei SaOS-2-Zellen, die auf der durch Coaten mit rekombinantem Schwamm-Kollagen Typ 3 und enzymatisch - mittels Silicatein-α und TEOS - synthetisiertem Biosilica modifizierter Oberfläche wuchsen (Well-Nr. 2A, 2B und 2C) sowie bei SaOS-2-Zellen, die auf der durch Coaten mit Rinder-Typ 1-Kollagen und enzymatisch - mittels Silicatein-α und TEOS - synthetisiertem Biosilica modifizierter Oberfläche wuchsen (Well-Nr. 3A, 3B und 3C) ein starker Anstieg der Mineralisierung gefunden.

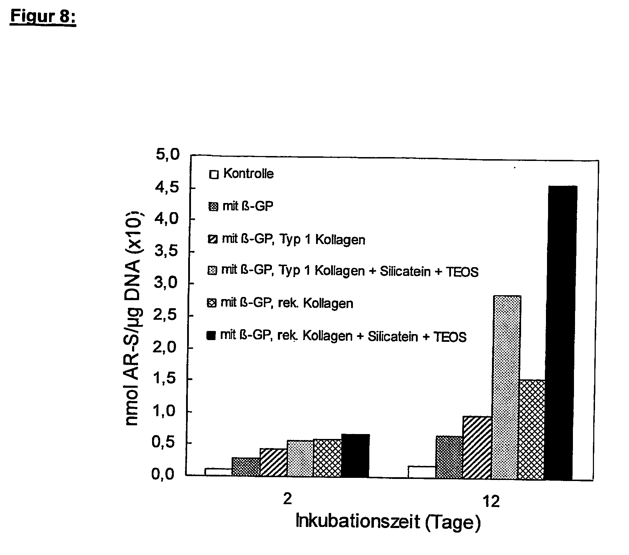

Der Anstieg der Mineralisierung von SaOS-2-Zellen, die auf der erfindungsgemäß enzymatisch modifizierten Oberfläche wuchsen (Behandlung mit Kollagen plus Silicatein-α plus TEOS) war drastisch erhöht im Vergleich zu SaOS-2-Zellen, die auf Oberflächen wuchsen, die mit Kollagen alleine modifiziert waren (Abbildung 8). Wie die Abbildung zeigt, lag am Tag 12 das Ausmaß der Mineralisierung (angegeben in nmol Alizarin Red/µg Gesamt-DNA) auf den Kulturplatten nach Coaten mit Typ 1 Kollagen plus Silicatein-α plus TEOS (Synthese von Biosilica-modifiziertem Rinder-Kollagen) bzw. nach Coaten mit rekombinantem Typ 3 Kollagen plus Silicatein-α plus TEOS (Synthese von Biosilica-modifiziertem Schwamm-Kollagen) deutlich über demjenigen der mit den jeweiligen Kollagenen alleine gecoateten Platten.

Die Bioaktivität der erfindungsgemäß enzymatisch modifizierten Oberflächen kann auch durch Messung der Aktivität der alkalische Phosphatase in mineralisierten Sa-OS-2-Zellen demonstriert werden.

### 2.2. Herstellung der Silicatein-Polypeptide

Die für die Modifikation des Kollagens benötigten Silicatein-Polypeptide können aus Geweben oder Zellen gereinigt oder rekombinant hergestellt werden.

### 2.2.1. Reinigung der Silicatein-Polypeptide aus natürlichen Quellen

Die Reinigung des Silicatein-α und Silicatein-β kann aus isolierten Spicula von Schwämmen durchgeführt werden.

### 2.2.2. Herstellung der rekombinanten Silicatein-Polypeptide

Die Herstellung der rekombinanten Proteine (Silicatein-α: SEQ ID No. 1; Silicatein-β: SEQ ID No. 3) kann in *E*. *coli* erfolgen. Auch die Herstellung in Hefen und Säugerzellen ist möglich. Hierzu wird die jeweilige cDNA in einen Expressionsvektor, z. B. *pQE-30,* einkloniert. Nach Transformation von *E*. *coli* wird die Expression der Proteine durch mit IPTG (isopropyl-β-D-thiogalactopyranosid) induziert (Ausubel et al. (1995) Current Protocols in Molecular Biology. John Wiley and Sons, New York). Die Aufreinigung der rekombinanten Proteine über das Histidin-Tag wird an einer Ni-NTA-Matrix durchgeführt.

Zwischen Oligohistidin und Silicatein kann eine Sequenz, die der Enterokinase-Spaltstelle entspricht, eingebracht werden. Das Fusionsprotein wird dann mit Enterokinase gespalten.

Alternativ kann zur Expression der rekombinanten Proteine z. B. das "GST (Glutathion-S-Transferase) Fusions"-Systems (Firma Amersham) benutzt werden. Es können zwei Inserte benutzt werden, um potentielle Effekte von Signalpeptiden während der Expression zu eliminieren; ein Insert umfasst das gesamte abgeleitete Protein (lange Form) und das andere Insert lediglich den aktiven Bereich (kurze Form). Die entsprechenden Klone werden in das Plasmid *pGEX-4T-2* eincloniert, welches das GST-Gen von *Schistosoma japonicum* enthält. Nach Transformation von *E*. *coli* wird die Expression der Proteine durch IPTG induziert. Die erhaltenen GST-Fusionsproteine werden durch Affinitätschromatographie an Glutathion-Sepharose 4B gereinigt. Zur Abtrennung der Glutathion-S-transferase werden die Fusionsproteine mit Thrombin gespalten.

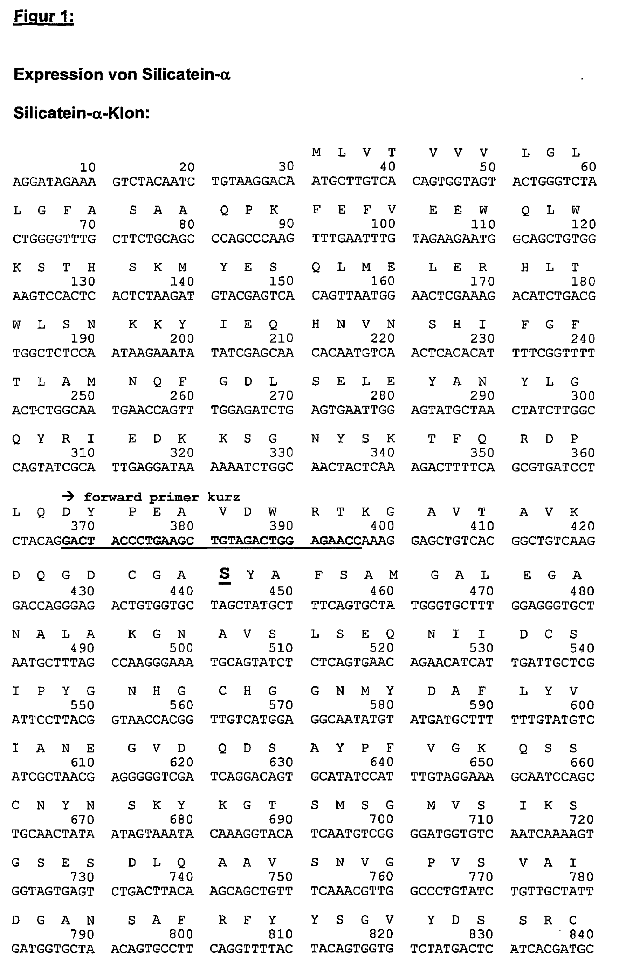

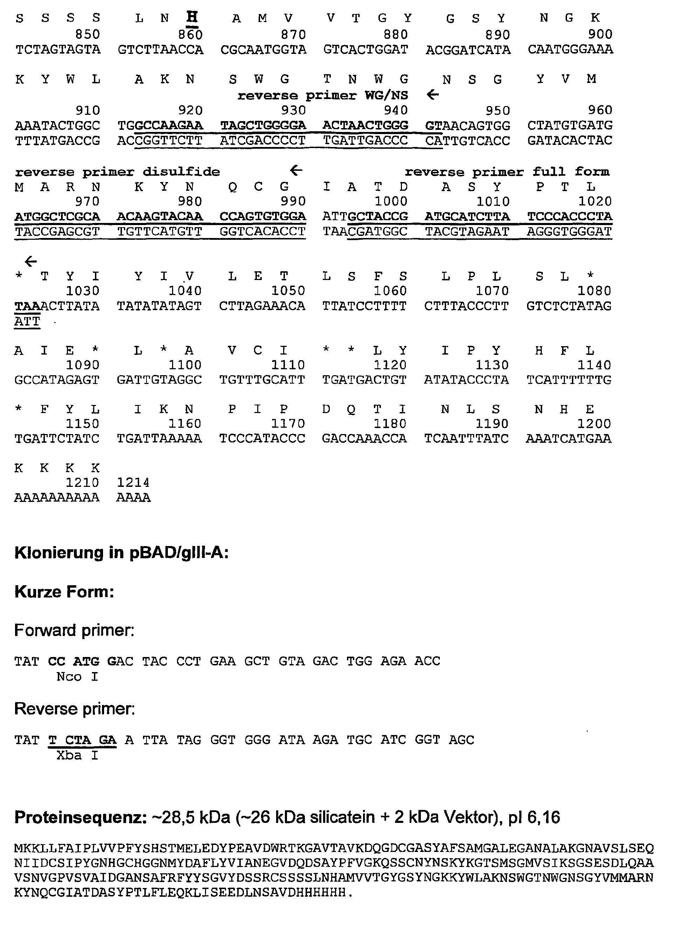

Eine weitere bevorzugte Alternative (benutzt für die hier beschriebenen Experimente) ist die Herstellung des rekombinanten Silicatein-α in *E*. *coli* unter Benutzung des Oligo-Histidin-Expressionsvektors pBAD/gIIIA (Invitrogen), bei dem auf Grund der Gen III-Signalsequenz das rekombinante Protein in den periplasmatischen Raum sezerniert wird (Figur 1). Die für das Silicatein-α kodierende cDNA-Sequenz (SEQ ID No. 2) wird mittels PCR unter Verwendung folgender Primer amplifiziert (kurze Form des Silicatein-α): Forward primer: TAT **CC ATG G**AC TAC CCT GAA GCT GTA GAC TGG AGA ACC (SEQ ID Nr. 9) und Reverse primer: TAT **T CTA GA** A TTA TAG GGT GGG ATA AGA TGC ATC GGT AGC (SEQ ID Nr. 10); und in pBAD/gIIIA einkloniert (Restriktionsnucleasen zur Insertion in den Expressionsvektor: *N*coI und *Xba*l)*.* Nach Transformation von *E*. *coli* XL1-Blue wird die Expression des Fusionsproteins mit L-Arabinose induziert.

Das rekombinante Schwamm-Silicatein-Polypeptid (kurze Form) besitzt ein Molekulargewicht von ∼28,5 kDa (∼26 kDa Silicatein plus 2 kDa Vektor) und einen isolelektrischen Punkt von pl 6,16.

Ebenso kann auch ein Insert benutzt werden, welches das gesamte abgeleitete Silicatein-α-Protein (lange Form) umfasst.

Auf analoge Weise kann eine kurze und eine lange Form des Silicatein-β (cDNA: SEQ ID No. 4; davon abgeleitete Aminosäuresequenz: SEQ ID No. 3) exprimiert werden.

### 2.3. Bestimmung der Silicatein-Aktivität

Zur Bestimmung der Enzymaktivität der (rekombinanten) Silicateine kann ein Assay angewandt werden, der auf der Messung von polymerisiertem und präzipitiertem Silica nach Hydrolyse und nachfolgender Polymerisation von Tetraethoxysilan (TEOS) basiert (Figur 3). Hierbei wird das Enzym üblicherweise in 1 ml eines MOPS-Puffers (pH 6,8) gelöst und mit 1 ml einer 1 - 4,5 mM Tetraethoxysilan-Lösung versetzt. Die enzymatische Reaktion wird für verschieden lange Zeiten üblicherweise bei Raumtemperatur durchgeführt. Zum Nachweis der Silica-Produkte wird das Material abzentrifugiert, mit Ethanol gewaschen und luftgetrocknet. Anschließend wird das Sediment mit 1 M NaOH hydrolysiert. In der entstandenen Lösung wird unter Anwendung eines Molybdat-gestützten Nachweisverfahrens (Silicon-Assay von Fa. Merck) das freigesetzte Silicat quantitativ gemessen.

Die Hydrolyse von Alkoxysilanen durch die (rekombinanten) Silicateine kann auch mit Hilfe eines gekoppelten optischen Tests bestimmt werden. Dieser Test beruht auf der Bestimmung des freigesetzten Alkohols. Hierzu werden in eine Küvette eine ABTS [Azino-bis (3-Ethylbenzthiazolin-6-sulfonsäure)]-Lösung in Kaliumphosphat-Puffer pH 7,5 (O₂-gesättigt) sowie eine Peroxidase- und eine Alkoholoxidase-Lösung pipettiert. Nach dem Mischen erfolgt die Zugabe von H₂O₂. Nach erneutem Mischen wird die Substratlösung (z.B. Tetraethoxysilan [TEOS] in MOPS-Puffer) bzw. das Enzym (Silicatein) in Substratlösung hinzugegeben und in einem Photometer die Extinktion bei 405 nm verfolgt. Zur Erstellung der Eichgerade dienen verschiedene Alkohol (z.B. Ethanol)-Konzentrationen.

### 2.4. Herstellung der Silicase aus natürlichen Quellen und es rekombinanten Enzyms

Die Reinigung der Silicase aus natürlichen Quellen (wie Gewebe oder Zellen) und die rekombinante Herstellung des Enzyms (SEQ ID No. 5) sind Stand der Technik (DE 102 46 186.4; WO 2004033679).

### 2.5. Bestimmung der Silicase-Aktivität

Die Methode zum Nachweis der Silicase-Aktivität von (kommerziellen) Carboanhydrasen-Präparationen (z. B. aus Rinder-Erythrozyten; Firma Calbiochem) bzw. der rekombinanten Schwamm-Silicase wurde beschrieben (DE 102 46 186.4; (WO 2004033679).

### 2.6. Herstellung von Schwammkollagen

Sowohl natives Kollagen (aus Vertebraten wie beispielsweise Rinderkollagen sowie aus Invertebraten (wie beispielsweise aus marinen Demospongien) als auch rekombinantes Kollagen (insbesondere aus dem marinen Schwamm *S*. *domuncula)* kann als Template benutzt werden. Im folgenden werden einige Verfahren zu ihrer Darstellung beschrieben.

### 2.6.1. Isolierung von nativem Schwamm-Kollagen

Ein einfaches Verfahren zur Isolierung von Kollagen aus verschiedenen marinen Schwämmen wurde beschrieben (DE 100 10 113 A 1**.** Verfahren zur Isolierung von Schwammkollagen sowie Herstellung von nanopartikulärem Kollagen. Anmelder: W. Schatton. Erfinder: Kreuter J, Müller WEG, Schatton W, Swatschek D, Schatton M; Swatschek et al. (2002) Eur J Pharm Biopharm 53:107-113). Das Schwammkollagen wird dabei mit hoher Ausbeute (> 30%) erhalten.

### 2.6.2. Herstellung von rekombinantem Schwamm-Kollagen

Zur Herstellung des rekombinanten Kollagens (SEQ ID No. 7) kann ein Klon verwendet werden, der für ein nichtfibrilläres Kollagen (Kollagen 3) aus *S*. *domuncula* codiert.

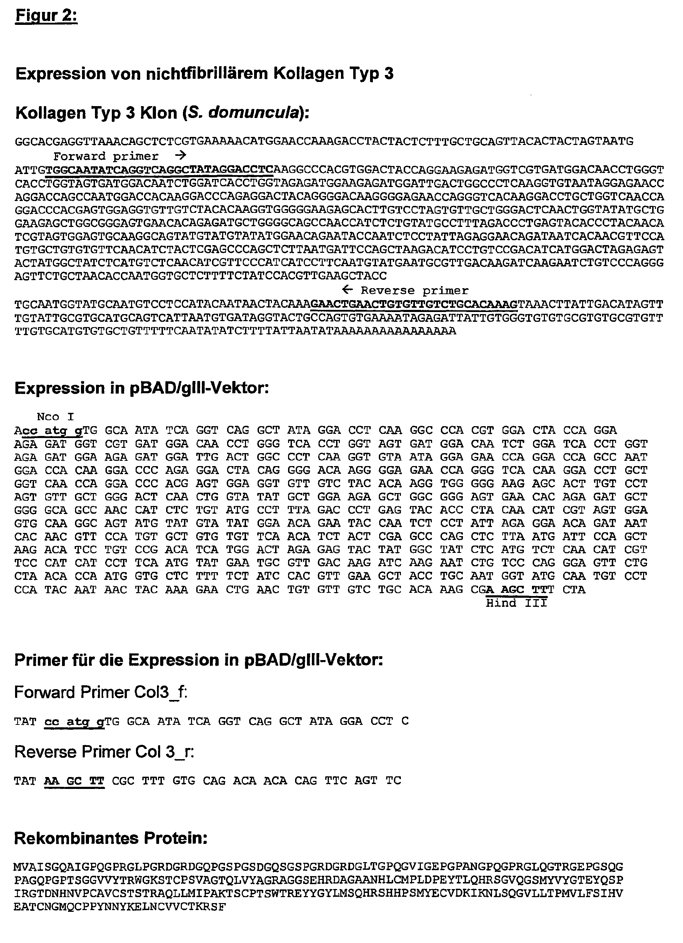

Die für das *S*. *domuncula* Typ 3 Kollagen kodierende cDNA-Sequenz (SEQ ID No. 8) kann mittels PCR unter Verwendung geeigneter Primer amplifiziert und in einen geeigneten Expressionsvektor subkloniert werden. Mit Erfolg wurde die Expression unter anderem mit den bakteriellen Oligo-Histidin-Expressionsvektoren pBAD/gIIIA (Invitrogen) und pQTK_1 (Qiagen) durchgeführt (Figur 2). Als Primer für die PCR (bei anschließender Verwendung von pBAD/gIIIA) können dienen; Forward primer: TAT **cc atg g** TG GCA ATA TCA GGT CAG GCT ATA GGA CCT C (SEQ ID No. 11) und Reverse primer: TAT **AA GC TT** CGC TTT GTG CAG ACA ACA CAG TTC AGT TC (SEQ ID No. 12); Restriktionsnucleasen zur Insertion in den Expressionsvektor: *N*coI und *Hin*dIII. Nach Transformation von *Escherichia coli*-Stamm XL1-Blue mit dem Plasmid (Expressionsvektor) wird die Expression des Fusionsproteins mit L-Arabinose (bei pBAD/gIIIA) bzw. mit Isopropyl-β-D-thiogalactopyranosid (IPTG; bei pQTK_1) induziert. Der Expressionsvektor pBAD/gIIIA besitzt den Vorteil, dass auf Grund der Gen III-Signalsequenz das rekombinante Protein in den periplasmatischen Raum sezerniert wird. Die Signalsequenz wird nach der Membran-Passage entfernt. Bei Verwendung von pQTK_1 werden die Bakterien mit PBS/8 M Harnstoff extrahiert. Nach Ultrabeschallung wird die Suspension zentrifugiert. Die Reinigung des Fusionsproteins aus dem Überstand erfolgt durch Metall-Chelat-Affinitätschromatographie unter Benutzung einer Ni-NTA-Agarose-Matrix (Qiagen), wie von Hochuli et al. (J Chromatogr 411:177-184; 1987) beschrieben. Der Extrakt wird auf die Säule gegeben; anschließend wird mit PBS/Harnstoff gewaschen und das Fusionsprotein von der Säule mit 150 mM Imidazol in PBS/Harnstoff eluiert.

Das Molekulargewicht des rekombinanten Typ 3 Kollagens *(S. domuncula),* das nach Expression der unter Benutzung der oben genannten Primer amplifizierten cDNA erhalten wird, beträgt - 28,5 kDa. Der isoelektrische Punkt (IEP) des aus der SEQ ID No. 8 gezeigten cDNA abgeleitete Peptids (siehe SEQ ID No. 7) ist 8,185. Die Ladung bei pH 7,0 beträgt 4,946.

### 2.7. Zellkultur

Humane Osteosarcomzellen (SaOS-2; American Type Culture Collection) werden in McCoy's Medium (Invitrogen), enthaltend 15% foetales Rinderserum (FBS) mit 1% Glutamin, 100 U/ml Penicillin, 100 µg/ml Streptomycin bei 37°C, 98-100% relativer Luftfeuchtigkeit und 5% CO₂-Atmosphäre kultiviert. Das Medium wird alle 2 Tage gewechselt. Zur Herstellung der experimentellen Kulturen werden die konfluenten Zellen kurz mit Hank's Balanced Saline Solution (HBSS) ohne Ca²⁺ und Mg²⁺ (Sigma) gewaschen und dann trypsinisiert; Behandlung mit 0,1 Gew.-% Trypsin / 0,04 Gew.-% EDTA in Ca²⁺ und Mg²⁺-freiem PBS (137 mM NaCl, 2,7 mM KCI, 10 mM Na₂HPO₄ 1,76 mM KH₂PO₄, pH 7,40). Nach der Bildung einer Zellsuspension wurden die Zellen in einem Hämocytometer gezählt und mit einer Dichte von 1000 Zellen/mm² in 24 Weil-Platten (190 mm²) ausgesät. Danach werden die Kulturen für bis zu 14 Tage in Wachstumsmedium inkubiert. Das Medium wurde alle 2 Tage und nach einer Woche jeden Tag gewechselt. Am Tag 7 wurde 10 mM β-Glycerophosphat (Sigma; 1 M Stocklösung) hinzugegeben. Durch β-Glycerophosphat wird die Mineralisierung stimuliert.

### 2.8. Behandlung der Kulturplatten und Durchführung des Assay

Die Kulturplatten werden mit PBS alleine (Kontrolle) oder Lösungen der folgenden Proteine in PBS gecoated:
(a) Typ 1 Kollagen (Sigma; 10 µg/cm²) plus Silicatein (1 µg/cm²) plus TEOS (5 mM) und
(b) Typ 3 Kollagen (10 µg/cm²) plus Silicatein (1 µg/cm²) plus TEOS (5 mM).

Hierzu werden die Mikrotiterplatten nach Zugabe von Kollagen, Silicatein und TEOS bei 37°C für 1 Stunde inkubiert. Danach werden die Platten mit PBS einmal gewaschen und die Zellen eingesetzt.

Auch andere Konzentrationen der Proteine und des Substrats sowie andere Inkubationszeiten haben sich als geeignet erwiesen.

Die Konzentration des rekombinanten Typ 3 Kollagens *(S. domuncula)* in der Stocklösung (PBS, filtriert) beträgt 400 µg/ml. Diese Lösung wurde zum Coaten (10 µg/cm²) 1 : 10 in PBS verdünnt.

Die Konzentration des Typ 1 Kollagens von Sigma in der Stammlösung (0,1 N Essigsäure, neutralisiert mit NaOH pH 7,0; filtriert) beträgt 400 µg/ml. Diese Lösung wurde zum Coaten (10 µg/cm²) 1 : 10 in PBS verdünnt.

Auch andere Konzentrationen des Kollagens und andere Kollagen-Typen haben sich als geeignet erwiesen.

Die Konzentration des rekombinanten Silicateins (Silicatein-α; *S*. *domuncula)* in der Stammlösung (PBS; filtriert) beträgt 40 µg/ml. Diese Lösung wird zum Coaten (1 µg/cm²) 1 : 10 in PBS verdünnt.

Auch andere Konzentrationen des Silicateins haben sich als geeignet erwiesen. Ebenso wie Silicatein-α kann auch Silicatein-β als Enzym zu Modifikation eingesetzt werden.

Die Stocklösung von Tetraethylorthosilicat (Tetraethoxysilan, TEOS; Aldrich) hatte eine Konzentration von 5 mM. TEOS wird in Dimethylsulfoxid in einer Stammlösung von üblicherweise 500 mM gelöst und anschließend zu der gewünschte Endkonzentration herunter verdünnt.

Auch andere Konzentrationen von TEOS und andere Substrate (Kieselsäuren, Monoalkoxysilantriole, Dialkoxysilandiole, Trialkoxysilanole oder Tetraalkoxysilane zur Herstellung von Silica und Monoalkoxysilandiole, Monoalkoxysilanole, Dialkoxysilanole, Alkyl-, Aryl- oder Metallo-Silantriole, Alkyl-, Aryl- oder Metallo-Silandiole, Alkyl-, Aryl- oder Metallo-Silanole, Alkyl-, Aryl- oder Metallo-Monoalkoxysilandiole, Alkyl-, Aryl- oder Metallo-Monoalkoxysilanole, Alkyl-, Aryl- oder Metallo-Dialkoxysilanole, Alkyl-, Aryl- oder Metallo-Trialkoxysilane zur Herstellung von Siliconen) haben sich als geeignet erwiesen.

### 2.9. Bestimmung der DNA-Konzentration

Die Gesamt-DNA in den Ansätzen lässt sich mit Hilfe von Methoden, die Stand der Technik sind, bestimmen, beispielsweise dem PicoGreen-Assay. Hierzu wird PicoGreen dsDNA Quantitation Reagent (Molecular Probes) 1:200 in TE-Puffer (10 mM Tris/HCl pH 7,4, 1 mM EDTA) verdünnt. Danach wird die PicoGreen-Lösung 1:1 (100 µl : 100 µl) mit den Proben (Zellen suspendiert in TE-Puffer) gemischt. Die Ansätze werden 5 Minuten im Dunkeln stehen gelassen und dann mit Hilfe eines Fluoreszenz-ELISA-Platten-Reader (z. B. Fluoroskan Version 4.0) bei einer Exzitation von 485 nm und Emission von 538 nm gemessen. Als Vergleichsstandard wird eine Eichkurve mit Kalbsthymus-DNA aufgenommen.

### 2.10. Nachweis der Mineralisierung mit Alizarin red S

Die Bildung von Calciumphosphat durch Osteoblasten wie z. B. SaOS-2-Zellen kann nach der Methode von Stanford et al. (J Biol Chem 270:9420-9428, 1995) oder anderen Methoden, die Stand der Technik sind, gemessen werden. Die Zellen werden 1 Stunde bei 4°C in 100%igem Ethanol fixiert, dann kurz mit destilliertem H₂O gewaschen und mit 40 mM Alizarin Red-S-Lösung (pH 4,2; Firma Sigma) 10 Minuten bei Raumtemperatur unter leichtem Schütteln gefärbt. Danach werden die Zellen mehrmals mit destilliertem H₂O und mit 1xPBS (DULBECCO) gewaschen. Danach werden die Zellen in 100 µl/cm² von 10 Gew.-% Cetylpyridiniumchlorid (CPC), 10 mM Natriumphosphat (pH 7,0) für 15 Minuten bei Raumtemperatur unter leichtem Schütteln inkubiert. Von den Überständen wird jeweils ein Aliquot 10-fach in 10% CPC, 10 mM Natriumphosphat (pH 7,0) verdünnt und die Absorption bei 562 nm gemessen. Die Mole gebundenes Alizarin Red-S können anhand einer Eichkurve bestimmt werden. Die erhaltenen Werte werden auf die in Parallel-Kulturen bestimmten Gesamt-DNA-Mengen bezogen.

### 3. Verwendungen

Weiterhin aufgeführt werden die nachstehend genannten Verwendungen des Verfahrens gemäß Anspruch 1 sowie der erhaltenen Produkte.
1. Verwendung des Verfahrens durch enzymatische Modifikation von Molekülen oder Molekülaggregaten auf Oberflächen mit amorphem Siliciumdioxid (Silica) sowie der erhaltenen Produkte in der Zellkultur, beim Tissue Engineering oder in medizinischen Implantaten.
2. Verwendung des Verfahrens sowie der erhaltenen Produkte zur Steigerung des Wachstums (von Zellen und Zellkulturen im allgemeinen und insbesondere von Fibroblasten und Knochen-bildenden Zellen/Osteoblasten) sowie der Steigerung der Mineralisierung (von Knochen-bildenden Zellen/Osteoblasten).
3. Verwendung des Verfahrens sowie der erhaltenen Produkte zur Erzeugung einer Matrix, welche die Ablagerung von Calciumphosphat bzw. Apatit begünstigt oder fördert.
4. Verwendung des Verfahrens sowie der erhaltenen Produkte zur Erzeugung einer stabilen Verbindung insbesondere zwischen Knochen und Implantaten, wobei es (a) zur Wanderung von Ca²⁺ und PO₄³-Gruppen aus der Lösung, dem Medium oder einer Körperflüssigkeit oder freigesetzt von Zellen oder gebildet unter Beteiligung zellulärer Enzyme (wie z. B. der Freisetzung von Phosphat aus β-Glycerophosphat mit Hilfe der Osteoblastenmembran-assoziierten alkalischen Phosphatase) an die SiO₂-Schicht auf der Oberfläche unter Ablagerung von Calciumphosphat kommt, (b) zum Wachstum der entstandenen amorphen Calciumphosphat-Schicht durch Einbau von weiterem löslichem Calcium und Phosphat und (c) zur Kristallisation der amorphen Calciumphosphat-Schicht durch Einbau von Hydroxid-, Carbonat- und FluoridAnionen (enthalten z. B. in aus Körperflüssigkeiten) unter Bildung eines gemischten, aus Hydroxyl-, Carbonat-, Fluorapatit bestehenden Apatitmaterials.
5. Verwendung des Verfahrens sowie der erhaltenen Produkte zur Verbesserung der Biokompatibilität von medizinischen Implantaten.
6. Verwendung des Verfahrens zur Herstellung von Beschichtungen für Biomaterialien, Kunststoffe, Metalle, Metalloxide und andere Materialien zur Förderung der Zelladhäsion an diese Materialien als eine Vorraussetzung für die Gewebeintegration mit der Oberfläche von Implantaten.
7. Verwendung des Verfahrens zur Herstellung von SiO₂-Schichten auf Oberflächengebundenen Molekülen oder Molekülaggregaten von Implantaten zur Herabsetzung immunologischer Reaktionen des Empfängerorganismus wie Antigen-Antikörperreaktionen oder Bindung von Komponenten des Komplementsystems an die Implantat-Oberfläche.

## Patentansprüche

1. Verfahren zur Unterstützung des Wachstums, der Aktivität und/oder der Mineralisierung von Zellen und/oder Zellkulturen ex vivo umfassend
a) Herstellung einer bioaktiven Oberfläche durch enzymatische Modifikation mit amorphem Siliciumdioxid und/oder Siliconen, umfassend eine Modifikation einer Oberfläche durch Coaten mit Kollagen und enzymatisch mittels eines Polypeptids, wobei das Polypeptid eine tierische, bakterielle, pflanzliche oder Pilz Silicatein-α- oder Silicatein-β-Domäne umfasst, die mindestens 50% Sequenzidentität zu der in SEQ ID Nr. 1 oder SEQ ID Nr. 3 gezeigten Sequenz aufweist, und wobei das Polypeptid eine Silicatein-Aktivität aufweist, und
b) in Kontakt bringen der Zellen und/oder Zellkulturen mit der erhaltenen bioaktiven Oberfläche.

2. Verfahren nach Anspruch 1, **dadurch gekennzeichnet, dass** als Substrat zur enzymatischen Modifikation Kieselsäuren, Monoalkoxysilantriole, Dialkoxysilandiole, Trialkoxysilanole oder Tetraalkoxysilane eingesetzt werden.

3. Verfahren nach Anspruch 1, **dadurch gekennzeichnet, dass** als Substrat zur enzymatischen Modifikation Monoalkoxysilandiole, Monoalkoxysilanole, Dialkoxysilanole, Alkyl-, Aryl- oder Metallo-Silantriole, Alkyl-, Aryl- oder Metallo-Silandiole, Alkyl-, Aryl- oder Metallo-Silanole, Alkyl-, Aryl- oder Metallo-Monoalkoxysilandiole, Alkyl-, Aryl- oder Metallo-Monoalkoxysilanole, Alkyl-, Aryl- oder Metallo-Dialkoxysilanole, Alkyl-, Aryl- oder Metallo-Trialkoxysilane eingesetzt werden.

4. Verfahren nach einem der Ansprüche 1 bis 3, wobei es sich bei der Oberfläche um die Oberfläche von Glas, Metallen, Metalloxiden, Kunststoffen, Biopolymeren oder anderen Materialien handelt.

5. Verfahren nach einem der Ansprüche 1 bis 4, wobei das Polypeptid des Silicatein-α- oder Silicatein-β aus *Suberites domuncula* gemäß SEQ ID Nr. 1 bzw. SEQ ID Nr. 3 oder ein Polypeptid, das in der Aminosäuresequenz der Silicatein-α- oder Silicatein-β-Domäne mindestens 50% Sequenzidentität zu der in SEQ ID Nr. 1 bzw. SEQ ID Nr. 3 gezeigten Sequenz besitzt und eine Silicatein-Aktivität aufweist, in einem Zellextrakt oder -lysat oder in gereinigter Form zur Verfügung gestellt wird.

6. Verfahren zur Unterstützung des Wachstums, der Aktivität und/oder der Mineralisierung von Zellen und/oder Zellkulturen nach Anspruch 1, wobei die Zellen Osteoblasten sind.

7. Verwendung eines Verfahrens nach einem der Ansprüche 1 bis 5 bei der Zellkultur, beim Tissue Engineering oder bei der Herstellung von medizinischen Implantaten.

## Claims

1. Method for promoting the growth, the activity and/or the mineralization of cells and/or cell cultures, comprising
a) producing of a bioactive surface by enzymatic modification with amorphous silicon dioxide and/or silicones, comprising a modification of a surface by coating with collagen and the enzymatic use of a polypeptide, wherein said polypeptide comprises an animal, bacterial, plant or fungal silicatein-α- or silicatein-β-domain, having at least 50% sequence identity to the sequence as shown in SEQ ID No. 1 or SEQ ID No. 3, and wherein said polypeptide exhibits a silicatein-activity, and
b) contacting of the cells and/or cell cultures with the bioactive surface so obtained.

2. Method according to claim 1, **characterized in that** silicic acids, monoalkoxy silanetrioles, dialkoxy silanedioles, trialkoxy silanoles or tetraalkoxy silanes are used as substrate for the enzymatic modification.

3. Method according to claim 1, **characterized in that** monoalkoxy silandioles, monoalkoxy silanoles, dialkoxy silanoles, alkyl-, aryl- or metallo-silanetrioles, alkyl-, aryl- or metallo-silanedioles, alkyl-, aryl- or metallo-silanoles, alkyl-, aryl- or metallo-monoalkoxy silanedioles, alkyl-, aryl- or metallo-monoalkoxy silanoles, alkyl-, aryl- or metallo-dialkoxy silanoles, alkyl-, aryl- or metallo-trialkoxy silanes are used as substrate for the enzymatic modification.

4. Method according to any of claims 1 to 3, wherein the surface is the surface of glass, metals, metal oxides, plastics, biopolymers or other materials.

5. Method according to any of claims 1 to 4, wherein said polypeptide of silicatein-α or silicatein-β from *Suberites domuncula* according to SEQ ID No. 1 or SEQ ID No. 3 or a polypeptide, that in the amino acid sequence of the silicatein-α- or silicatein-β-domain exhibits at least 50% sequence identity to the sequence as shown in SEQ ID No. 1 or SEQ ID No. 3, and having a silicatein activity, is provided in a cell extract or lysate or in purified from.

6. Method for promoting the growth, the activity and/or the mineralization of cells and/or cell cultures according to claim 1, wherein the cells are osteoblasts.

7. Use of a method according to any of claims 1 to 5 in cell culture, tissue engineering or the production of medical implants.

## Revendications

1. Procédé pour la stimulation de la croissance, de l'activité et/ou de la minéralisation de cellules et/ou de cultures cellulaires ex vivo comprenant
a) la production d'une surface bioactive par une modification enzymatique avec du dioxyde de silicium amorphe et/ou des silicones, comprenant une modification d'une surface en la revêtant de collagène et de manière enzymatique au moyen d'un polypeptide, le polypeptide comprenant des domaines de silicatéine α ou de silicatéine β d'origine animale, bactérienne, végétale ou provenant de champignons, qui présentent au moins une identité de séquence de 50 % avec la séquence présente dans la SEQ ID N°1 ou la SEQ ID N°3, et où le polypeptide présente une activité de silicatéine, et
b) la mise en contact des cellules et/ou des cultures cellulaires avec les surfaces bioactives obtenues.

2. Procédé selon la revendication 1 **caractérisé en ce qu'**en tant que substrat pour les modifications enzymatiques on emploie des acides siliciliques, des monoalcoxysilanetriols, des dialcoxysilanediols, des trialcoxysilanols ou des tétraalcoxysilanes.

3. Procédé selon la revendication 1 **caractérisé en ce qu'**en tant que substrat pour des modifications enzymatiques des monoalcoxysilanediols, des monoalcoxysilanols, des dialcoxysilanols, des alkyl-, des aryl-silanetriols ou des silanetriols métalliques, des alkyl-, des aryl-monoalcoxysilanediols ou des monoalcoxysilanediols métalliques, des alkyl-, des aryl-silanols ou des silanols métalliques, des alkyl-, des aryl-monoalcoxysilanediols ou des monoalcoxysilanediols métalliques, des alkyl-, des aryl-monoalcoxysilanols ou des monoalcoxysilanols métalliques, des alkyl-, des aryl-dialcoxysilanols ou des dialcoxysilanols métalliques, des alkyl-, des aryl-trialcoxysilanes ou des trialcoxysilanes métalliques sont employés.

4. Procédé selon l'une des revendications de 1 à 3 où, en tant que surface, il s'agit de verre, de métaux, d'oxydes métalliques, de matières synthétiques, de biopolymères ou d'autres matériaux.

5. Procédé selon l'une des revendications de 1 à 4 où le polypeptide de silicatéine α ou de silicatéine β provenant de *Suberites domuncula* selon SEQ ID N°1, respectivement SEQ ID N°3, ou un polypeptide qui possède dans la séquence des acides aminés de la silicatéine β ou de la silicatéine β des domaines au moins à 50 % d'identité de séquence avec la séquence montrée dans SEQ ID N° 1, respectivement dans SEQ ID N°3, et présente une activité de silicatéine, est mis à la disposition dans un extrait cellulaire ou un lysat cellulaire ou dans une forme purifiée.

6. Procédé pour la stimulation de la croissance, de l'activité et/ou de la minéralisation de cellules et/ou de cultures cellulaires selon la revendication 1 où les cellules sont des ostéoblastes.

7. Utilisation d'un procédé selon l'une des revendications de 1 à 5 dans la culture cellulaire, dans l'ingénierie des tissus ou dans la production d'implants médicinaux.
